# EUROPÄISCHE PATENTSCHRIFT

(11) **EP 0 873 104 B1**
(45) Veröffentlichungstag und Bekanntmachung des Hinweises auf die Patenterteilung: **08.09.2004**
(21) Anmeldenummer: 96946120.1
(22) Anmeldetag: 27.12.1996
(51) Int. Cl.: A61H 9/00

(54) **VERFAHREN UND VORRICHTUNG ZUR NICHTINVASIVEN ENTNAHME VON KÖRPERFLÜSSIGKEIT UND KÖRPERREINIGUNG**
PROCESS AND DEVICE FOR NON-INVASIVELY DRAWING OFF BODY FLUID AND BODY CLEANING
PROCEDE ET DISPOSITIF POUR LE PRELEVEMENT NON INVASIF DE LIQUIDE ORGANIQUE ET LE NETTOYAGE DU CORPS

(30) Priorität: 29.12.1995 DE 19549316; 04.07.1996 DE 29612291 U
(43) Veröffentlichungstag der Anmeldung: 28.10.1998
(73) Patentinhaber: Manukow, Sarkis, Dr., 14469 Potsdam (DE)
(72) Erfinder: MANUKOW, Sarkis, D-14467 Potsdam (DE); BRISKER, Wiktor, D-14471 Potsdam (DE)
(74) Vertreter: Hengelhaupt, Jürgen, Dipl.-Ing.
(86) Internationale Anmeldenummer: PCT/DE1996/002515
(87) Internationale Veröffentlichungsnummer: WO 1997/024100

(56) Entgegenhaltungen:
- EP-A- 0 330 472
- WO-A-92/22276
- DE-U- 8 330 191
- DE-U- 8 811 822
- DE-U- 9 204 198
- GB-A- 2 160 444

## Beschreibung

Die Erfindung betrifft ein Verfahren und eine Vorrichtung zur nichtinvasiven Entnahme von Körperflüssigkeit durch die Haut und ist anwendbar insbesondere zum Transport von flüssigen Substanzen in der bzw. durch die menschliche Haut während einer zeitlich definierten Einwirkung ohne irreversible Veränderungen der Haut.

Die Erfindung beschreibt weiterhin eine Vorrichtung zur Reinigung des menschlichen Körpers und ist anwendbar insbesondere im häuslichen Bereich sowie im Bereich der Krankenpflege sowie der Seniorenbetreuung.

Aufgabe der menschlichen Haut ist es, einen Informations- und Stoffluß in beiden Richtungen der Haut zu kontrollieren und zu koordinieren, wobei die Ausführung dieser Aufgaben den streng reglementierten biophysikalischen und biochemischen Barrieren ausgesetzt ist. In den Fällen, wenn die Notwendigkeit besteht, einen definierten Informations- und/oder Stoffluß in den oder aus dem menschlichen Körper zu transportieren, müssen solche Barrieren überwunden werden.

Es ist aus der Fachliteratur bekannt, über spezielle Applikationen an der Haut einen Flüssigkeitstransport in eine der Hautrichtungen zu ermöglichen. Die Palette dieser Beispiele reicht von den aus der Volksmedizin stammenden Methoden wie Sauna, warme und kalte Feuchtekompressen (Temperatureinwirkung), Einsatz von Saugflaschen und Schropfköpfen (Negativdruck) bis zu modernen Methoden hin, vor allem aus dem physiotherapeutischen Bereich, die auf der Einwirkung des elektromagnetischen Feldes (UHF-Feld, Elektrophorese, die IR-Strahlung) beruhen.

Da bei diesen Methoden mit nur einem physikalischen Faktor gewirkt wird, kann man die komplex gesetzten Funktionsbarrieren der behandelten Körperbereiche schwer überwinden oder es sind die zu erzwingenden Prozesse zeitlich und räumlich schwer steuerbar.

Nachteilig an diesen bekannten Lösungen ist ebenfalls, daß der Einsatz dieser Methoden außerdem stark auf sehr spezifische Problemlösungen eingeschränkt ist und kaum Querverbindungsmöglichkeiten für andere Zwecke bietet. Zu dieser Gruppe lassen sich auch spezielle Methoden zuordnen, die durch Einwirkung eines gewählten physikalischen Faktors auf nichtinvasiver weise einen Transportvorgang durch die Haut ermöglichen. Ein komplexes Zusammenwirken mehrerer physikalischer Größen wurde bisher nicht ermöglicht bzw. angeregt.

Aus der EP -A- 0 330 472 ist eine Vorrichtung zur Erzeugung von Temperaturmustern mit in benachbarten Bereichen unterschiedlichen Temperaturen auf der Hautoberfläche eines Patienten bekannt, bei der die Temperaturmuster sequentiell veränderbar sind. Zur Vorgabe der Temperaturmuster und Steuerung ihrer sequentiellen Wechsel ist dabei eine elektrische Schaltung mit einem programmierbaren Microcomputer vorgesehen.

Es ist weder eine Druckeinheit noch eine Elektroeinheit vorhanden. Die Entnahme von Körperflüssigkeiten ist nicht vorgesehen.

Weiterhin ist aus der DE -U- 8 330 191 eine Vorrichtung zum Waschen von Personen bekannt, bei welcher heißes und kaltes Wasser über ein Mischventil zu warmen Wasser gemischt und das warme Wasser der Hautoberfläche über eine Wasserzuleitung und Düsen zugeführt und über eine mit Unterdruck beaufschlagte Wasserableitung wieder abgesaugt wird. Eine Thermokontrastierung der Hautoberfläche erfolgt nicht.

Mit der WO 92/22276 wird.schließlich eine Körperreinigungsvorrichtung beschrieben, welche ebenfalls das zugeführte Reinigungsmittel wieder absaugt, wobei die Auslassöffnung für das Reinigungsmittel in einem Gehäuse im Abstand von der zu reinigenden Körperfläche angeordnet ist, und das Gehäuse an seinem äusseren Rand eine Dichtung zur Anlage an die Körperfläche aufweist und die Einlassöffnungen der Absaugung am körpernahen Ende des Gehäuses in der Gehäusewand angeordnet sind.

Der Erfindung liegt daher die Aufgabe zugrunde, ein Verfahren und Vorrichtungen zu entwickeln, welche es auf einfachem Wege, zuverlässig, gesundheitlich unbedenklich und preiswert gestatten, die Hautschicht als biophysikalische und biochemische Schutzschicht des menschlichen Körpers zeitweilig durchlässig zu machen und einen kontrollierten Transportvorgang von flüssigen Stoffen in einer der beiden Hauptrichtungen zu unterstützen, bei dem die gewünschte Menge des jeweiligen Stoffes, ohne die Haut im Wirkungsprozeß irreversibel zu zerstören, durch die Hautschicht überführt werden kann.

Der Erfindung liegt weiterhin die Aufgabe zugrunde, eine Vorrichtung zu schaffen, mit welcher der menschliche Körper effektiv und zuverlässig gereinigt werden kann, ohne daß die Reinigungsflüssigkeit an nicht dafür vorgesehene Körperstellen sowie in das Umfeld der Person gelangt und die Handbrause einfach anwendbar und preiswert herstellbar ist.

Im Vergleich zu den bekannten Verfahren besteht der Vorteil des erfindungsgemäß vorgeschlagenen Verfahrens darin, daß gezielt, vom Verhalten der Körperzellen in Abhängigkeit von unterschiedlichen physikalischen Einflußfaktoren ausgehend, eine zeitgleiche komplexe Wirkung ausgewählter Faktoren vorgenommen wird, wodurch alle Schichten des Hautsystems über die Initiierung der gas-flüssigen Konturen der Körperflüssigkeit über die gesamte Hauttiefe in den Wirkungsbereich einbezogen werden. Insbesondere zeichnet sich aber das erfindungsgemäß vorgeschlagene Verfahren gegenüber vielen herkömmlichen Methoden dadurch aus, daß die Einwirkungsmechanismen grundsätzlich ohne irreversible Zerstörung des Hautsystems ablaufen, indem eng beianderliegende kleinste Bereiche der Hautoberfläche abwechselnd zeitgleich warm und kalt beaufschlagt werden derart, daß warme und kalte Bereiche aneinandergrenzen und im vorgegebenen Zeittakt umgepolt werden, wobei zusätzlich zu der Warm-Kalt-Wechselbeaufschlagung der Hautoberfläche eine zeitlich definierte Wechselbeaufschlagung mit elektrischem Strom und/oder Über- bzw. Unterdruck möglich ist.

Desweiteren ist es vorteilhaft möglich, über die Anzahl der einwirkenden Faktoren bzw. über derer gezielt gewählten Merkmale unterschiedliche Aufgaben ausüben zu können, die mit dem Transport bzw. der Zirkulation der Flüssigkeit im menschlichen Gewebe, vorwiegend aber in oberflächennahe liegenden Körperbereichen, zusammenhängen.

Ein weiterer Vorteil der Erfindung liegt in einer wirtschaftlichen gerätetechnischen Realisierung des Verfahrens, indem in einem Gehäuse mindestens ein Thermoblock und mindestens ein Elektroblock und/oder mindestens ein Druckblock angeordnet ist, und die einzelnen Blöcke über eine Steuerung nach einem vorgebenen Programm aktiviert werden.
Damit sind gerätetechnisch Vorrichtungen zur nichtinvasiven Entnahme der Körperflüssigkeit und/oder Injektion von Fluiden, Thermokontrastduschen und Reinigungsvorrichtungen realisierbar.

Der besondere Vorteil der Reinigungsvorrichtung besteht darin, daß die aus dem Brausekopf austretende Reinigungsflüssigkeit im wesentlichen nur mit den Körperstellen in Kontakt kommt, wo dies zum Zwecke der Reinigung auch vorgesehen ist, indem die aus dem Brausekopf austretende Reinigungsflüssigkeit nach erfolgtem Hautkontakt und somit erfolgter Reinigung umgehend durch die mit einem Unterdruck beaufschlagten Abzugskanäle wieder abgesaugt wird, ohne sich auf dem Körper oder in die Umgebung ausbreiten zu können. Realisierungen sind als Handbrause oder zumindest teilweise automatisierte Brausezelle, wo Brauseköpfe über den Körper geführt oder Brausemodule mit dem Körper in Kontakt gebracht werden, möglich.

Die Erfindung soll nachstehend anhand von Ausführungsbeispielen näher erläutert werden.
Es zeigen:
- Fig. 1: eine zusammengestellte Prinzipdarstellung der drei Blöcke
- Fig. 2: ein Blockschaltbild zum Zusammenwirken der drei Blöcke
- Fig. 3: eine Detailansicht der Vorrichtung zur nichtinvasiven Entnahme der Körperflüssigkeit in Schnittdarstellung mit mäanderförmigem (m) und schlangenförmigem (s) Aufbau der Peltier-Elemente
- Fig. 4: eine Detailansicht der Thermokontrastkompresse in Schnittdarstellung mit mäander- und schlangenförmigem Aufbau der Peltier-Elemente
- Fig. 5: einen Flußplan für die Zu- und Abführungen der Thermokontrastdusche
- Fig. 6: eine Prinzipdarstellung der Thermokontrastdusche im Schnitt
- Fig. 7 und Fig. 8: Detailansichten eines Moduls der Thermokontrastdusche in Bezug auf die Verteilung des Kanalsystems

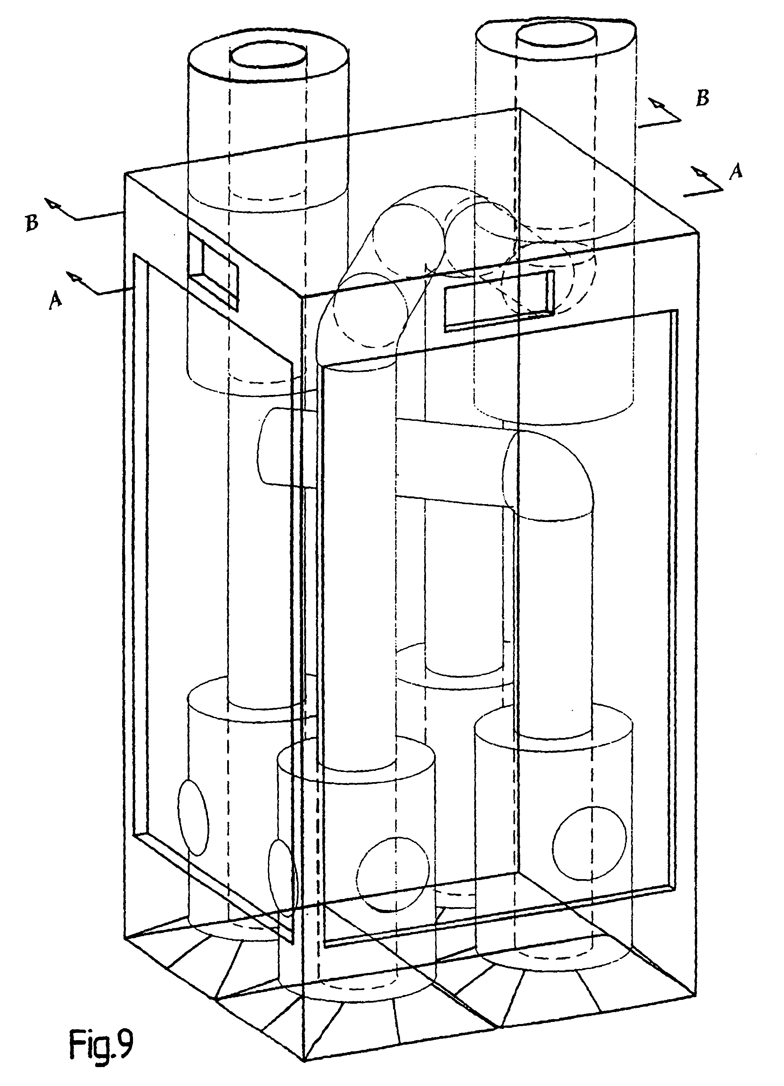

- Fig. 9: eine räumliche Darstellung eines Modules einer Thermokontrastdusche
- Fig. 10: eine Prinzipdarstellung einer Variante der Reinigungsmittelzuführung in Zuführungskanälen
- Fig. 11: eine Prinzipdarstellung einer Variante der Reinigungsmittelzuführung in Zuführungskanälen und der Reinigungsmittelabführung in Abzugskanälen
- Fig. 12: eine Prinzipdarstellung einer weiteren Variante der Reinigungsmittelzuführung in Zuführungskanälen und der Reinigungsmittelabführung in Abzugskanälen in Sicht auf die Ein- und Austrittsfläche
- Fig. 13: eine stilisierte Detailansicht eines Moduls mit Übereinanderanordnung der Zuführungs- und Abzugskanäle
- Fig. 14: eine räumliche Teildarstellung eines Moduls mit Minibad
- Fig. 15: eine Schnittdarstellung durch die Handbrause mit beweglich angeordneten Modulen
- Fig. 16a: eine Variante der Handbrause mit Zusatzteil mit Dichtring aus Hohlfasern und Ventilsteuerung
- Fig. 16b: eine Detaildarstellung einer Hohlfaser
- Fig. 16c: Gestaltungsvarianten der Fasern bzw. Manschetten
- Fig. 16d: Varianten der Befestigung und der Anordnung von Fasern bzw. Lamellen
- Fig. 17: ein Blockschaltbild für die Reinigungsmittelzubereitung mit Begasung
- Fig. 18: einen Flußplan für die Zu- und Abführungen einer Thermokontrastdusche
- Fig. 19: eine Prinzipdarstellung der Thermokontrastdusche im Schnitt
- Fig. 20 und Fig. 21: Detailansichten eines Moduls der Thermokontrastdusche in Bezug auf die Verteilung des Kanalsystems
- Fig. 22: eine räumliche Darstellung eines Modules einer Thermokontrastdusche

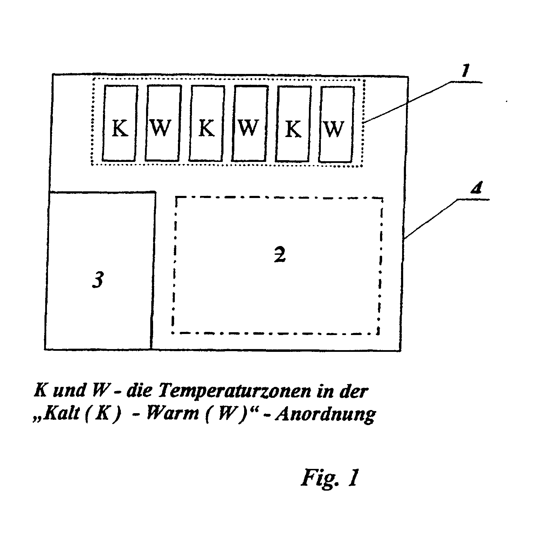

Wie in Figur 1 dargestellt, kann die erfindungsgemäß konzipierte Vorrichtung aus drei Blöcken, dem Thermoblock 1, dem Elektroblock 2 und dem Druckblock 3 bestehen, wobei alle drei Blöcke in einem Gehäuse 4 funktional gekoppelt und in der Weise angeordnet sein können, daß die thermische, elektrische und drucktechnische Einwirkung auf die in Kontakt eintretende Körperoberfläche ungehindert ausgeübt werden kann.

Nachfolgend wird eine Vorrichtung zur nichtinvasiven Entnahme der Körperflüssigkeit und/oder zur nichtinvasiven Injektion von Fluiden beschrieben.

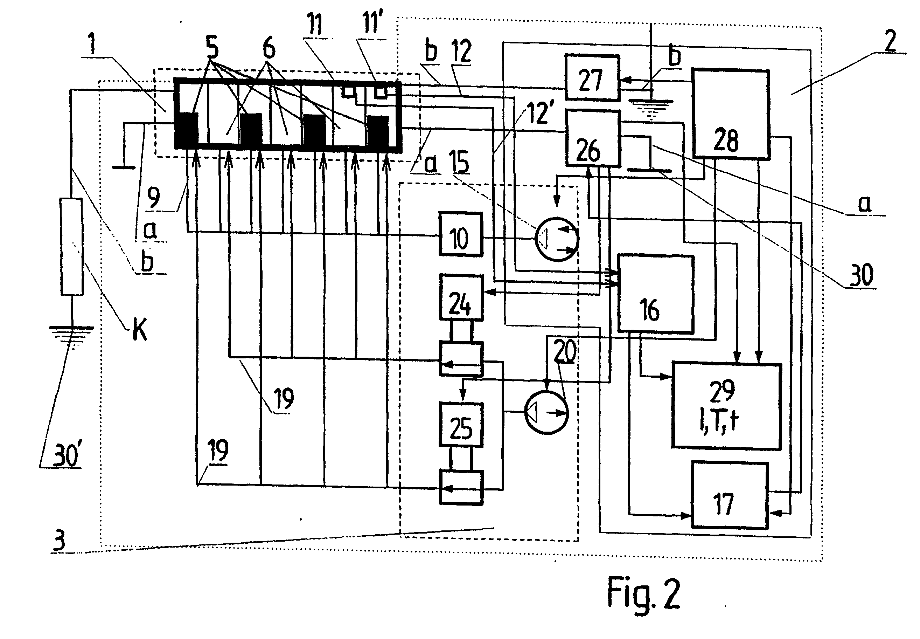

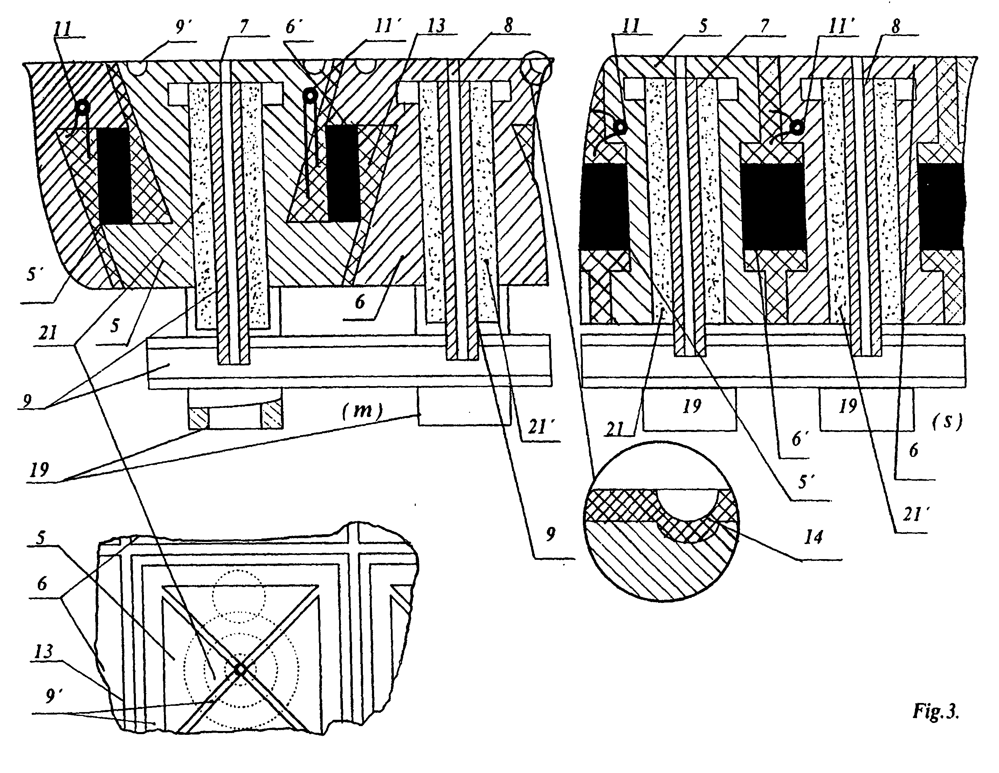

Die wesentlichen funktionalen Elemente des Thermoblockes 1 stellen die nach außen freigesetzten Temperaturzonen 5 und 6 dar, wie in Figur 2 und 3 zu ersehen ist. Die Temperaturzonen 5 und 6 werden konstruktiv durch wärme- und/oder elektroleitende Plättchen eines Thermoelementes, zum Beispiel eines mäanderförmigen (m) oder schlangenförmigen (s) Peltier-Elementes mit dazwischengeschalteten n- und p-Halbleiterschenkeln 5' und 6' gebildet. Die Gestalt und die Abmaße der Plättchen sind so gewählt, daß die Temperaturzonen nach außen gleichflächig und abwechselnd in der "Kalt-Warm"-Anordnung in einer Ebene ("Schachbrett"-Anordnung) liegen, während die Übergangsbereiche zwischen den Temperaturzonen 5 und 6 mit einem Isolierstoff 13 ausgefüllt sind. Die Außenseite, also die aktive Seite des Thermoelementes, ist mit einem wärme- und begrenzt stromleitenden Überzug 14 bedeckt.
Jedes Plättchen weist inmitten eine durchgehende Bohrung 7 und 8 auf, die über Kanalsysteme 9 und 9' mit dem dazu angeschlossenen Sammelreservoir 10 jeweils an eine der beiden doppelfunktionalen Pumpen 15 des Druckblockes 3 angeschlossen ist. In zwei Temperaturzonen 5 und 6 sind seitlich Thermosensoren, zum Beispiel Thermistoren 11 und 11' untergebracht, die über die Leitungen 12 und 12' mit einem Meßverstärker 16 und einer elektronischen Reguliereinheit 17 des Elektroblockes 2 verbunden sind.

Alle Temperaturzonen 5 und 6 des Thermoelementes sind an der Rückseite mit einem Kühlsystem 19 versehen, welches über die elektromechanischen Ventile 24 und 25 an eine Pumpe 20 angeschlossen ist und eine zeitlich und räumlich separate Kühlung der momentan warmeingeschalteten Bereiche über die in ihm integrierten porösen Kühlelemente 21 und 21' gewährleistet (vgl. Fig. 3).

Die Arbeitsweise der doppelfunktionalen Pumpen 15 wird über einen Taktgeber von dem Taktgeberblock 26 reguliert, so daß je nach Bedarf dadurch ein "positiver" oder ein "negativer" Druck im Kanalsystem 9 ausgeübt werden kann.
Der Elektroblock 2 besteht im wesentlichen aus zwei direkt funktional gekoppelten Konturen - die Gleichstromkontur (a) und die HF-Kontur (b), bestehend im wesentlichen aus einem HF-Generator, dem Thermoblock und dem momentan eingeschalteten menschlichen Körper k, sowie aus weiteren Hilfskonturen, die miteinander indirekt gekoppelt sind und die Versorgung von elektronischen und elektromechanischen Baugruppen wie Pumpen, Indikatoren und ähnlichem gewährleisten.
Die Funktion von (a) besteht in der von der Versorgungseinheit 28 durchzuführenden Versorgung des Thermoelementes. Zur Unterstützung der Funktion des Thermoelementes ist an ihm ein Kühlsystem 19, 20, 24, 25 mit einem vorgegebenen Programm gekoppelt, das über einen mit einer Steuerlogik ausgestatteten Taktgeber des Taktgeberblockes 26 das zeitliche Umschalten der Kontaktzonen reguliert. Zur Indikation der Ausgangssignale ist an 16 ein digitales Read-Out 29 angeschlossen.
Die Funktion von (b) beschränkt sich im wesentlichen auf die Generierung eines HF-Wechselstromes 27 der bei einem nachgeschalteten hochohmigen Widerstand sowie eines Stomstabilisators mit einer gewünschten Stromstärke über das Thermoelement auf den Körper k übertragen wird.

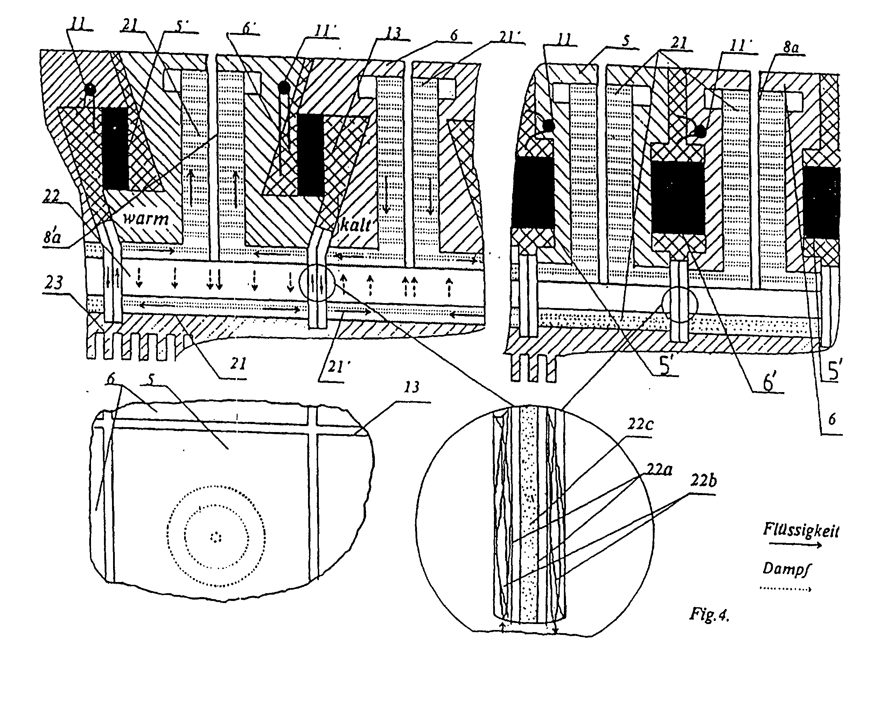

Nachfolgend soll zusätzlich eine Thermokontrastkompresse beschrieben werden. Die hierzu konzipierte Vorrichtung besteht in Analogie zu der Vorrichtung zur nichtinvasiven Entnahme der Körperflüssigkeit aus einem Thermoblock 1 und einem Elektroblock 2 mit Speisefunktion bei entfallenem Druckblock 3.
Der Thermoblock 1 stellt die zusammengestellten Module dar die in ihrer konstruktiven Ausführung analog zu der Vorrichtung zur nichtinvasiven Entnahme der Körperflüssigkeit die Temperaturzonen (Warm- und Kaltzonen) beinhalten, wie in Fig. 4 dargestellt. Im Unterschied zu der Vorrichtung zur nichtinvasiven Entnahme der Körperflüssigkeit ist bei dieser Vorrichtung ein Kanalsystem mit entsprechenden Bohrungen inmitten der Wärmezonen sowie ein an dieses System angeschlossenes Reservoir nicht explizit enthalten. Der Wärme- und der Stromtransport erfolgen angesteuert vom Elektroblock 2, im vorgegebenen Zeittakt ebenfalls über die Kontaktzonen. Die Kühlung des Thermoblockes 1 im Arbeitsregime wird entweder passiv allein über den kontaktierenden Körper oder passiv und transpirativ über den Körper und parallel dazu über ein geschlossenes transpiratives Kühlsystem 22 erfolgen. Dieses System 22 besteht aus einer Kanalleitung 8a' zur Dampf-Feuchte-Umwandlung, den zweischichtigen Diaphragmen aus einem Polymerfilm 22b mit einer darauf haftenden tränkfähigen Schicht 22a und einer hafthemmenden Pulvermenge 22c und sorgt dann dafür, daß die im entsprechenden Zeittakt aufgewärmten Temperaturzonen 5 bzw. 6 von dem gerippten großflächigen Kühlkörper 23 und von den unmittelbar an der Rückseite der Temperaturzonen 5 und 6 angebrachten porösen Kühlelementen 21 bis auf die gewünschte Temperatur gekühlt werden.
Der Elektroblock 2 enthält außer dem HF-Generator alle die in der Vorrichtung zur nichtinvasiven Entnahme der Körperflüssigkeit angegebenen Elemente.

Im folgenden wird eine Thermokontrastdusche mit Absaugung beschrieben.

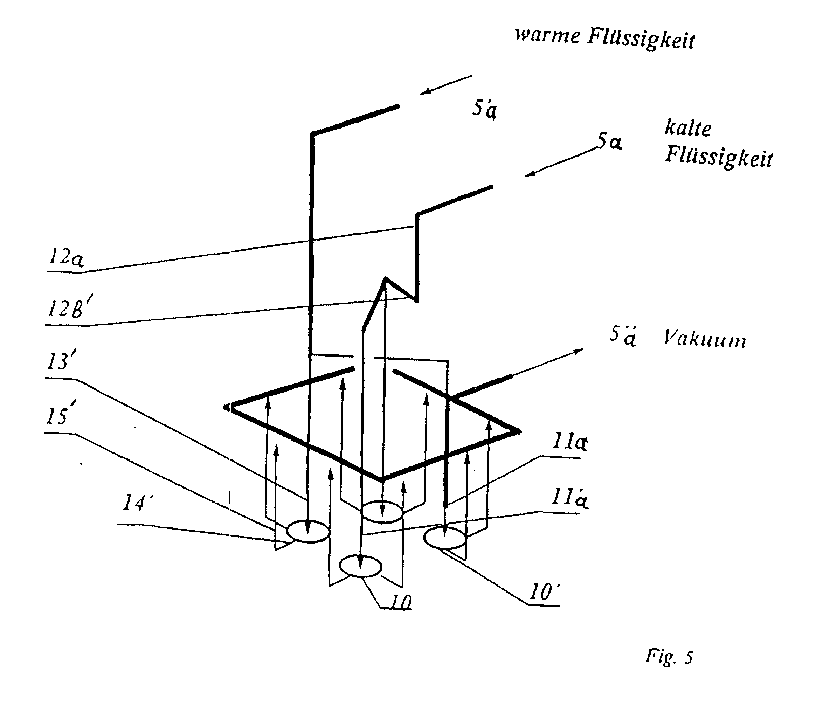

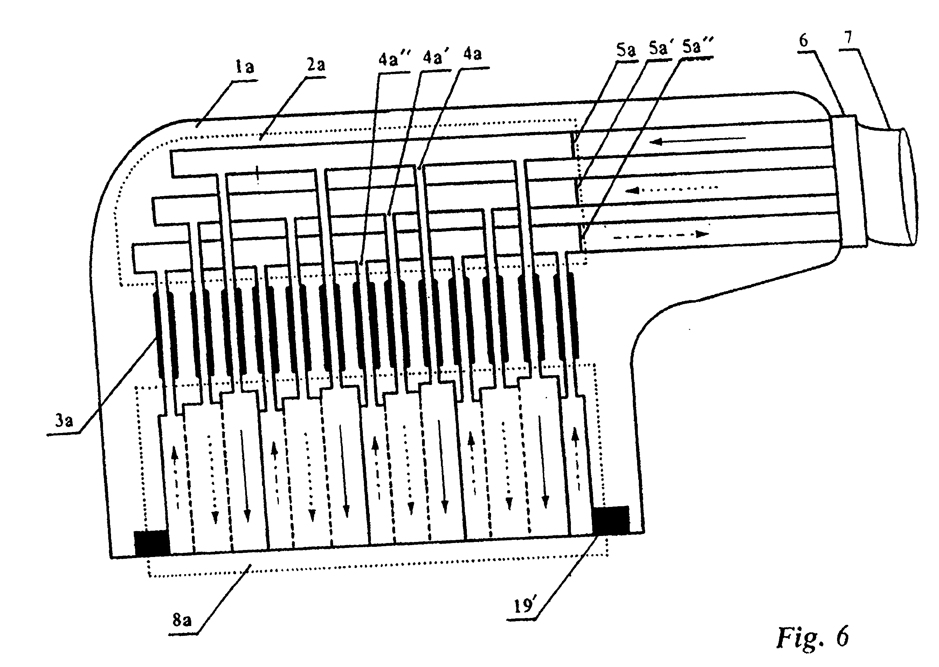

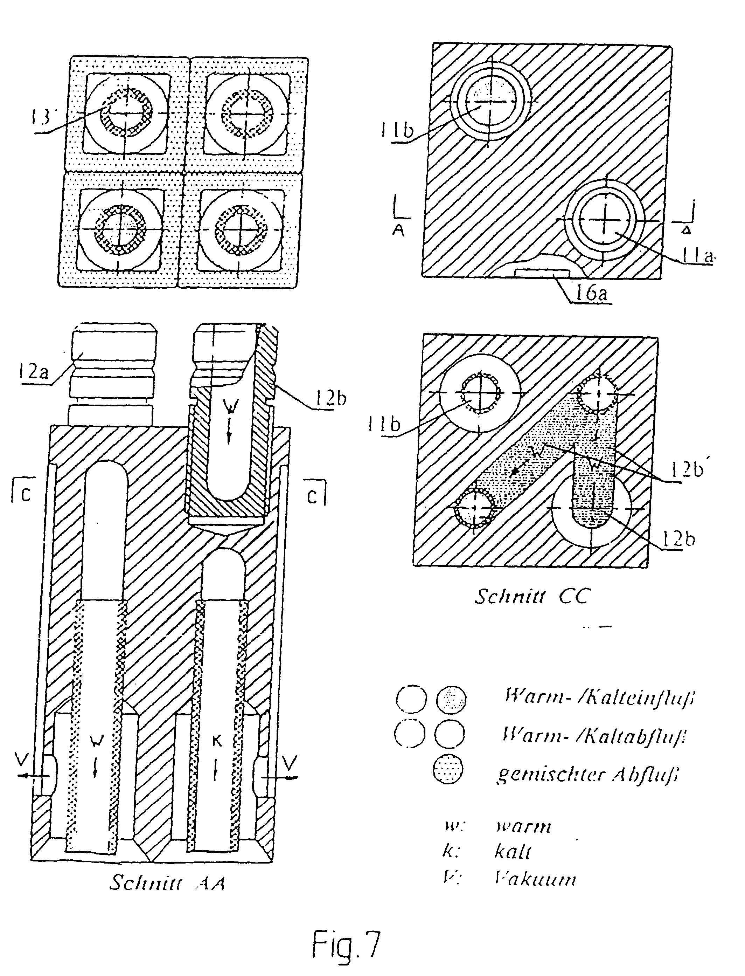

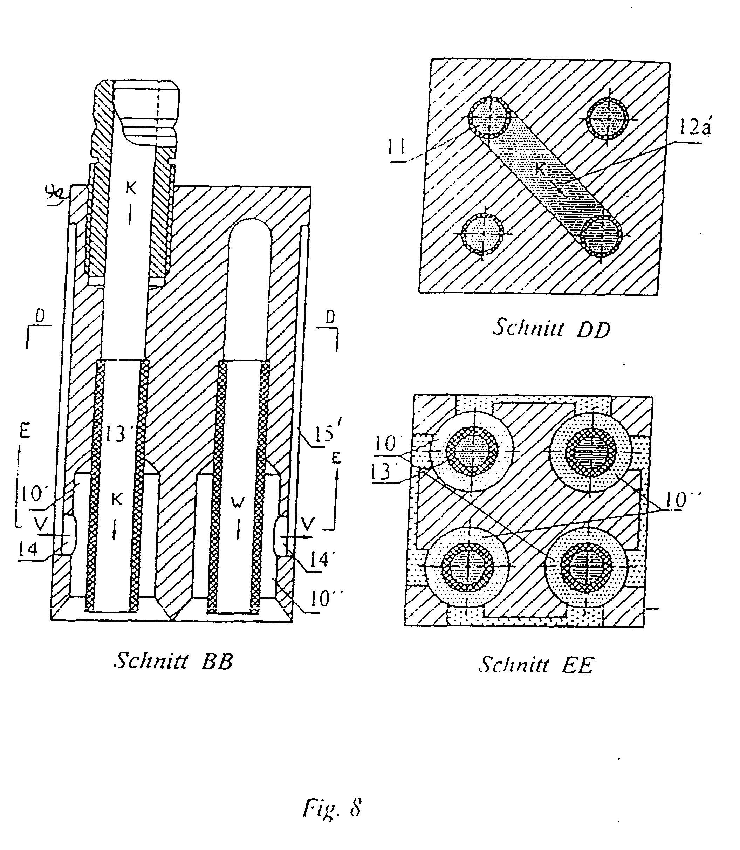

Die in den Figuren 5 bis 8 dargestellte Vorrichtung besteht aus einem Hohlkörper 1a mit unterschiedlich dimensionierten Ein- und Ausgängen. Am Ausgang werden zwei übereinander in einem Abstand angeordnete Aufsätze 2a und 8a montiert, die miteinander mit Verbindungsschläuchen 3a gekoppelt sind.
Der Aufsatz 2a ist an den Innenwandungen des Hohlkörpers 1a starr befestigt und weist in seinem Inneren zwei separate Kanalsysteme zum Durchfluß von fluiden Stoffen auf. Jedes der Kanalsysteme ist mit gleicher Anzahl der senkrecht zur Stirnseite des Aufsatzes durchgehenden Bohrungen 4a, 4a' und 4a" vom gleichen Querschnitt ausgestattet und derer Austrittsdüsen münden in einer Ebene der vorderen Stirnseite des Aufsatzes 2a ein. Die gegenüberliegenden Enden der Bohrungen 4a, 4a' und 4a" der jeweiligen Kanalteilsysteme münden über Modulzuleitungen in einem der beiden zentralen Leitungsnetze ein, während die Einmündungsflächen in drei verschiedenen Ebenen innerhalb des Aufsatzes 8a liegen. Die hintere Stirnseite des Aufsatzes 2a ist mit drei Bohrungen 5a, 5a' und 5a" größeren Querschnittes versehen, die über die zentralen Leitungsnetze mit den jeweiligen Kanalteilsystemen verbunden sind. Die Eingangsdüsen der Bohrungen 5a, 5a' und 5a" sind über die Flansche 6 mit dem Zentralschlauch 7 verbunden, über den eine entsprechende Versorgung mit fluiden Stoffen erfolgen kann.

Der Aufsatz 8a besteht aus den zusammengefügten, gegeneinander beweglichen Modulen 9a aus einem wärmeisolierenden Material, die mit einer gleichen Anzahl von durchgehenden Bohrungen gleichen Querschnittes versehen sind. Die Bewegungsfreiheit der Module gegeneinander ist durch die seitlich je zwei angeordneten Nuten 16a, in denen sich je eine rollende Kugel befindet, eingeschränkt.
Jeder der Module 9a weist eine Anzahl der senkrecht zu seiner Vorderseite angeordneten und etwa in der Mitte abgesetzten Bohrungen 10' und 10" auf, die je nach dem zuständigen Temperaturbereich über ein Kanalsystem 11a und 11b, zwei horizontale Kanalsysteme 12a' und 12a", an eine der beiden Zentralbohrungen 12 und 12' auf der Rückseite des Moduls angeschlossen sind. Im unteren Bereich (Vorderseite) haben die abgesetzten Bohrungen 10' und 10" einen größeren Durchmesser als hinter dem Absatz, der durch eine flache Abstufung ausgebildet ist. Jede dieser Bohrungen ist am Ausgang konisch unter einem Winkel von ca. 45° abgesetzt. In jede Bohrung wird ein Rohr 13' bis zum Anschlag an der Abstufung eingesetzt und befestigt, wobei seine Abmaße so gewählt sind, daß der Durchfluß des fluiden Stoffes bis zur Haut hin und der Abfluß des Stoffes über die vierkantigen Abzugskanäle 15 sich ungehindert vollziehen kann. Entlang der Seitenflächen jeden Moduls 9a sind vierkantige Vertiefungen bzw. Abzugskanäle 15' angeordnet, die unterhalb der Höhenabgrenzungen der Module liegen und über die Querkanäle 14' und 14" mit den Bohrungen 10' und 10" nach innen verbunden sind. Die Höhenlage der Abzugskanäle ist so gewählt, daß sie ein gemeinsames Abzugsnetz mit der Einmündung in einen zentralen Abzugskanal 5a" bilden, wodurch die Absaugung des fluiden Stoffes nach außen erfolgen kann.
Am Umfang des Aufsatzes 8a kann eine Manschette 19' aus einem wärme- und/oder feuchteisolierenden Stoff befestigt werden.

Nachfolgend wird die Reinigungsvorrichtung näher erläutert.

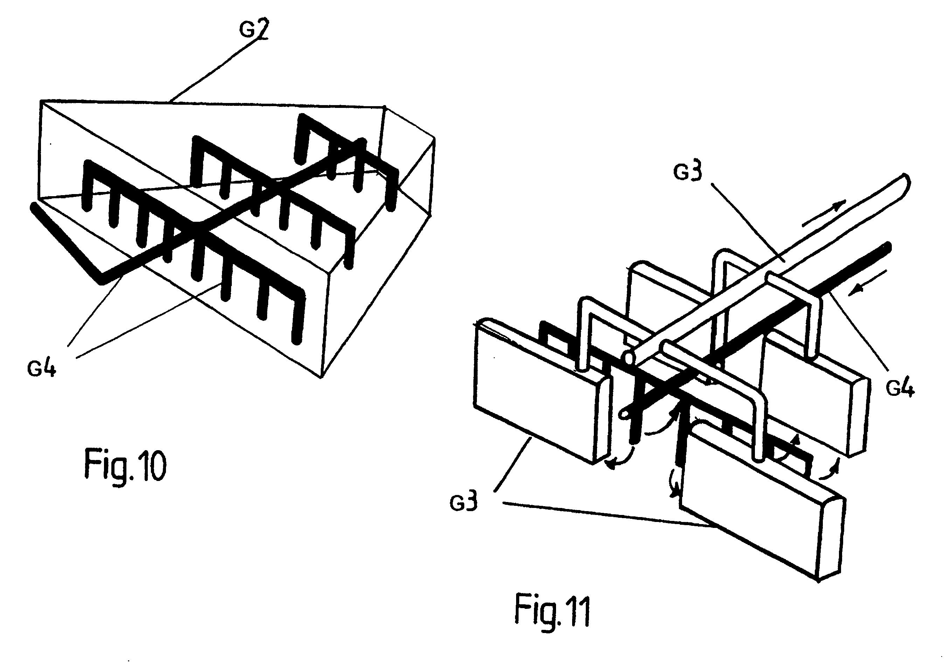

Wie aus Fig. 10 in Prinzipdarstellung ersichtlich ist, werden gemäß einer ersten Variante die Reinigungsmittel in Zuführungskanälen G4 dem Brausekopf G2 zugeführt. Das Reinigungsmittel ist im vorliegenden Ausführungsbeispiel speziell begastes Wasser. Die Konturen einer Handbrause sind stilisiert dargestellt, selbstverständlich sind runde oder kurvenförmig ausgebildete Ausführungsvarianten möglich. Ebenso ist es möglich, anstelle einer Handbrause eine Brausezelle zu realisieren, in welcher Brauseköpfe über den Körper geführt oder Brausemodule mit der Körperoberfläche in Kontakt gebracht werden.

In Fig. 11 ist zusätzlich zu den Zuführungskanälen G4 eine mögliche Anordnungsvariante der Abzugskanäle G3 zu ersehen, wobei die Abzugskanäle G3 über und neben den Zuführungskanälen G4 angeordnet sind.

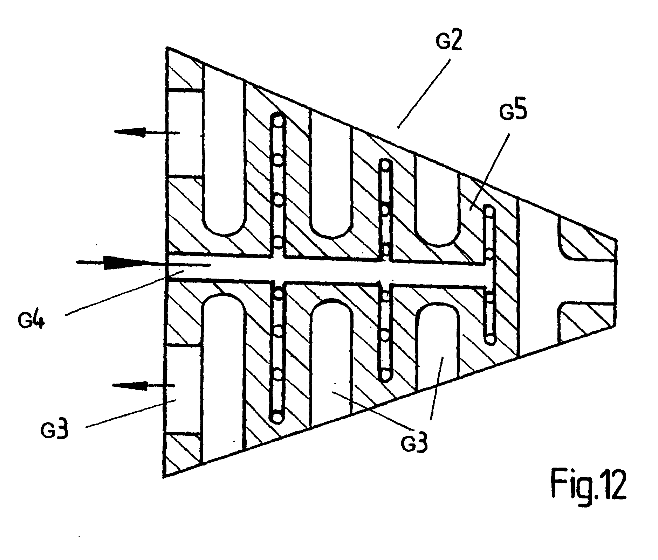

Fig. 12 zeigt eine Ansicht auf die Ein- und Austrittsfläche G5 eines Modules G15 des Brausekopfes G2 mit den Zuführungskanälen G4 und den Abzugskanälen G3.
Die Abzugskanäle G3 befinden sich zwischen den Zuführunskanälen G4 und sind an der Ein- und Austrittsfläche taschenförmig ausgebildet, so daß ein effektives Abpumpen der Reinigungsflüssigkeit erfolgt.

Ebenso ist es möglich, in den Figuren nicht dargestellt, die Module faltenbalgförmig zu gestalten derart, daß mehrere Faltenbalgkonstruktionen ineinandergeschachtelt angeordnet sind und hierdurch die Zuführungs- und Abzugskanäle bilden.

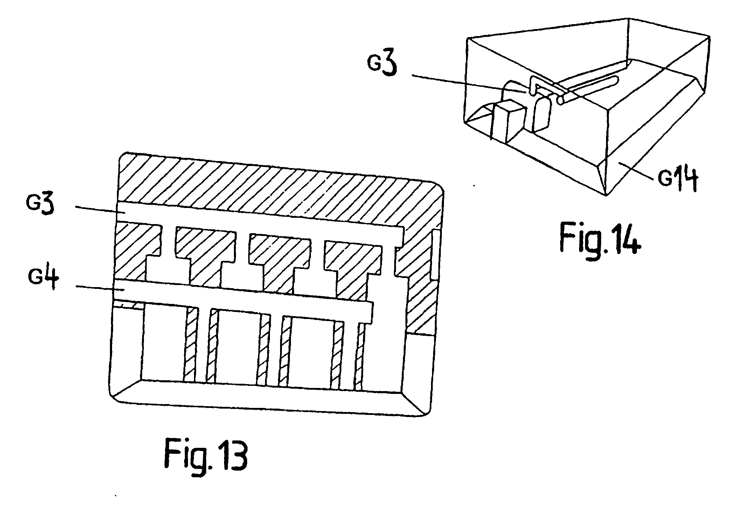

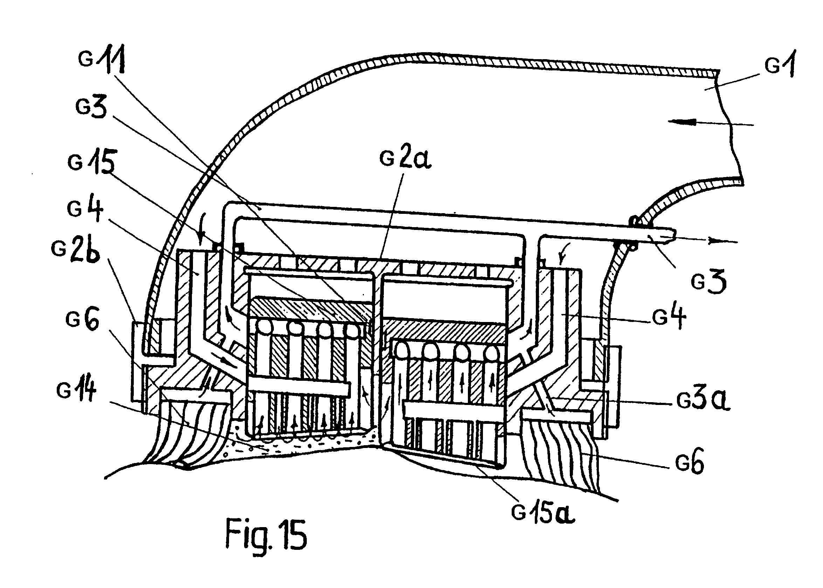

Aus Fig. 13 ist in einer stilisierten Detailansicht die Übereinanderanordnung der Abzugskanäle G3 über den Zuführungskanälen G4 zu ersehen. Fig. 14 ist eine räumliche Teildarstellung eines Moduls G15 und zeigt die Anordnung eines Minibades G14.

In Fig. 15 ist eine Ausführungsvariante dargestellt, bei welcher in einer herkömmlichen Handbrause ein Zusatzteil G2a eingesetzt ist, welches die Zuführungskanäle G4 und die Abzugskanäle G3 aufweist. Die Befestigung des Zusatzteiles G2a an der Handbrause erfolgt hier mittels einem Befestigungsring G2b.
Eine Besonderheit bei dieser Ausführungsvariante ist, daß die Module G15 beweglich ausgebildet sind derart, daß in Abhängigkeit der Stellung der Module G15 in senkrechter Richtung der Reinigungsmittelzufluß durch die Zuführungskanäle G4 entweder freigegeben oder abgesperrt ist. Im vorliegenden Ausführungsbeispiel ist der Reinigungsmittelzufluß zu dem Modul G15 über dem Minibad G14 freigegeben, der Zufluß an dem benachbarten Modul G15 jedoch verschlossen. Die Abzugskanäle G3 sind bei beiden Modulen G15 geöffnet. Die Unterseite G15a der Module G15 ist konisch ausgebildet.

Es ist jedoch ebenso möglich, an den Zuführungskanälen G4 und/oder den Abzugskanälen G3 Ventile anzuordnen, welche den Reinigungsmittelfluß steuern.
Der Dichtring G6 ist in diesem Ausführungsbeispiel als eine Manschette aus Fasern mit unterschiedlichem Durchmesser ausgebildet, wobei die Fasern hohl sind und über die Verbindungskanäle G3a mit den Abzugskanälen G3 verbunden sind.

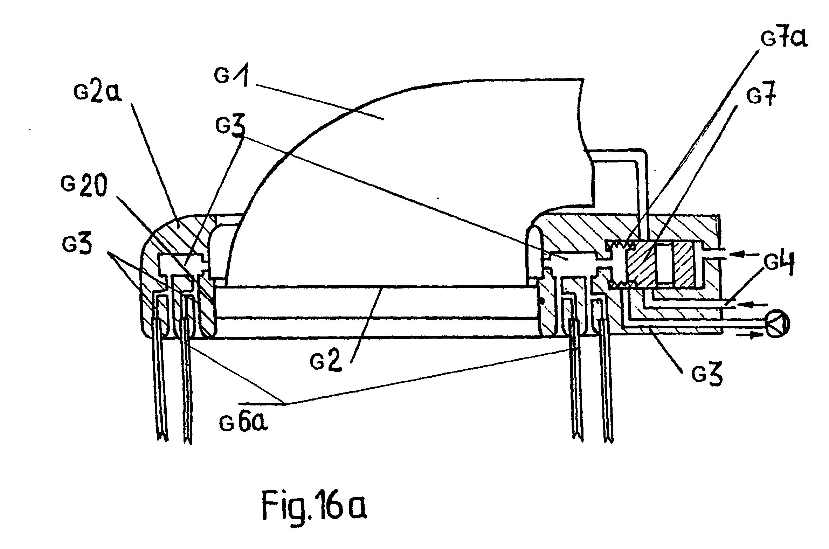

Eine weitere Ausführungsvariante der Handbrause ist in Fig. 16a dargestellt. Das Zusatzteil G2a ist hier über einen Befestigungs- und Höhenverstellungsring G20 an dem herkömmlichen Brausekopf G2 befestigt. An dem Zusatzteil G2a sind Hohlfasern G6a angeordnet, welche mit den Abzugskanälen G3 in Verbindung stehen und einerseits abdichten, andererseits zusätzlich absaugen. Eine Besonderheit dieser Ausführungsvariante ist das Ventil G7, welches die Zufuhr des Reinigungsmittels über die Zuführungskanäle G4 in Abhängigkeit des Unterdruckes in den Abzugskanälen G3 steuert. Bei fehlendem Unterdruck in den Abzugskanälen G3, also ohne Absaugung, ist die Zufuhr der Reinigungsmittel zur Hautoberfläche gesperrt. Mit zunehmendem Unterdruck wird die Reinigungsmittelzufuhr bis zu einem vorgegebenen Maximum freigegeben. Das Ventil G7 wirkt hierbei mit einer Feder G7a zusammen.

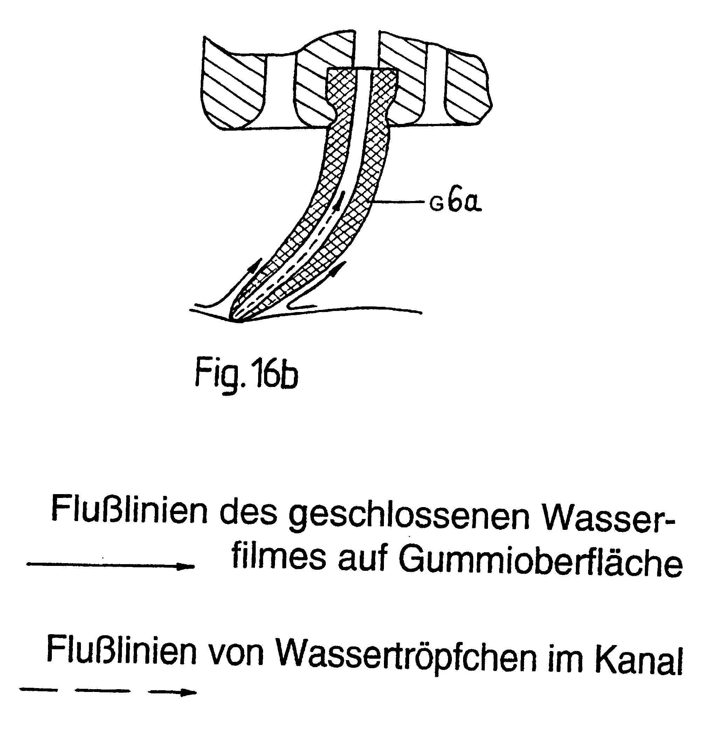

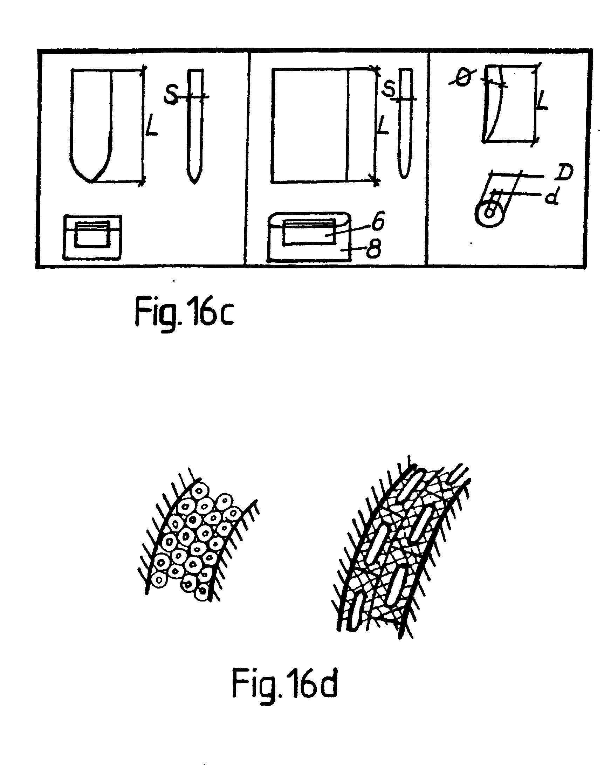

Fig. 16b zeigt eine Detailansicht einer Hohlfaser G6a während der Absaugung, in Fig. 16c und 16d sind Einzelheiten der Gestaltung und Anordnung der Fasern G6a bzw. Lamellen zu ersehen, wobei diese runde und/oder elyptische und/oder rechteckige Querschnitte aufweisen können.

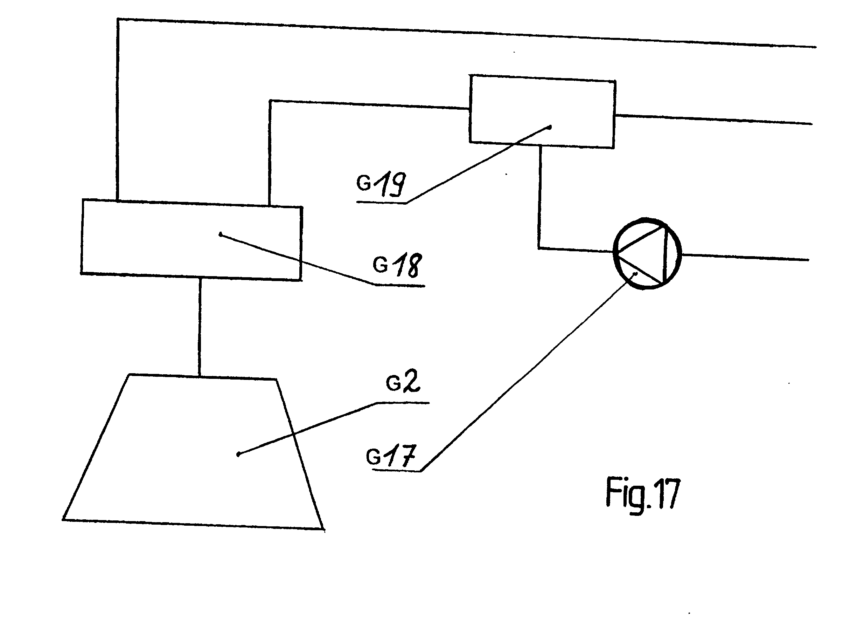

In Fig. 17 ist in Form einer Blockdarstellung eine mögliche Variante der Reinigungsmittelzubereitung gezeigt. Das zufließende warme und kalte Wasser wird in einer Mischbatterie G18 auf die gewünschte Mischtemperatur gebracht und dem Brausekopf G2 zugeführt. Zusätzlich wird über eine Begasungseinrichtung G19, im vorliegenden Ausführungsbeispiel im Kaltwasserstrang angeordnet, das Wasser begast. Die Begasungseinrichtung G19 ist mit einer Begasungspumpe G17 verbunden. Weiterhin ist es möglich, dem Reinigungsmittel über in der Fig. 17 nicht dargestellte Wege Behandlungsmittel hinzuzufügen oder das Reinigungsmittel direkt mittels spezieller Vorrichtungen zu behandeln.
Ebenso ist es möglich, an den in den übrigen Figuren dargestellten Zuführungsleitungen G4 Öffnungen zur Begasung anzuordnen, durch welche Gase, beispielsweise Luft, in das Reinigungsmittel injiziert werden.

Im folgenden wird eine modifizierte Variante einer Thermokontrastdusche mit Absaugung beschrieben.

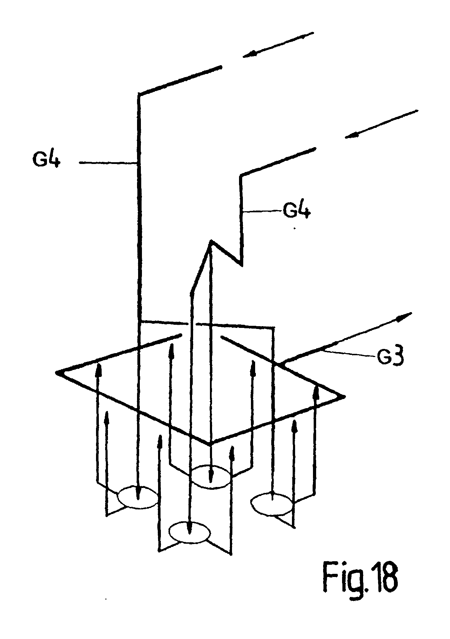

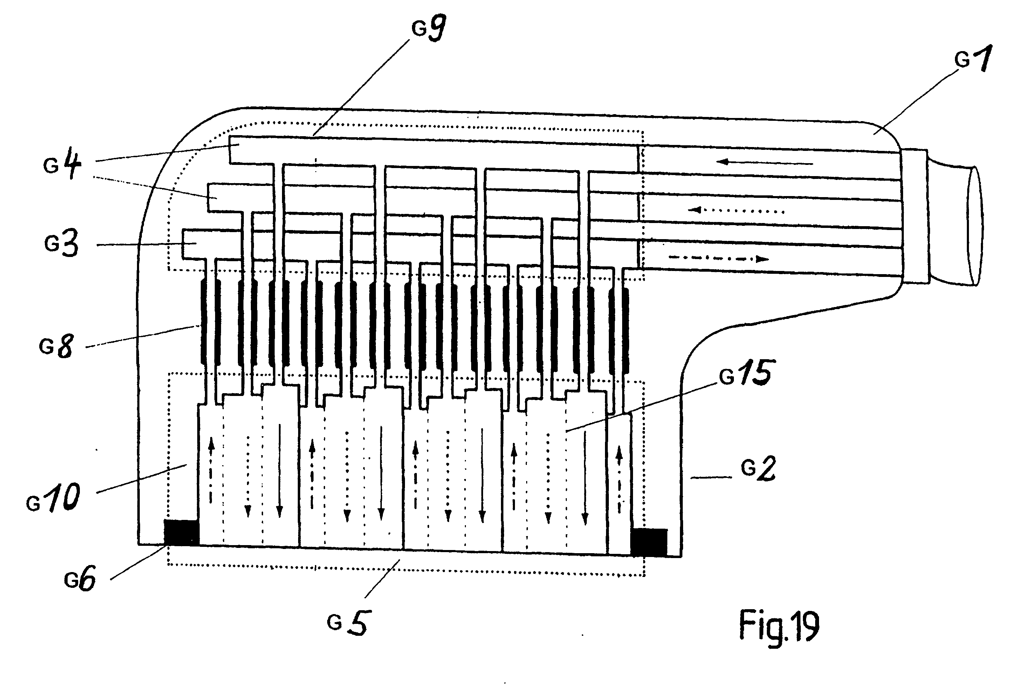

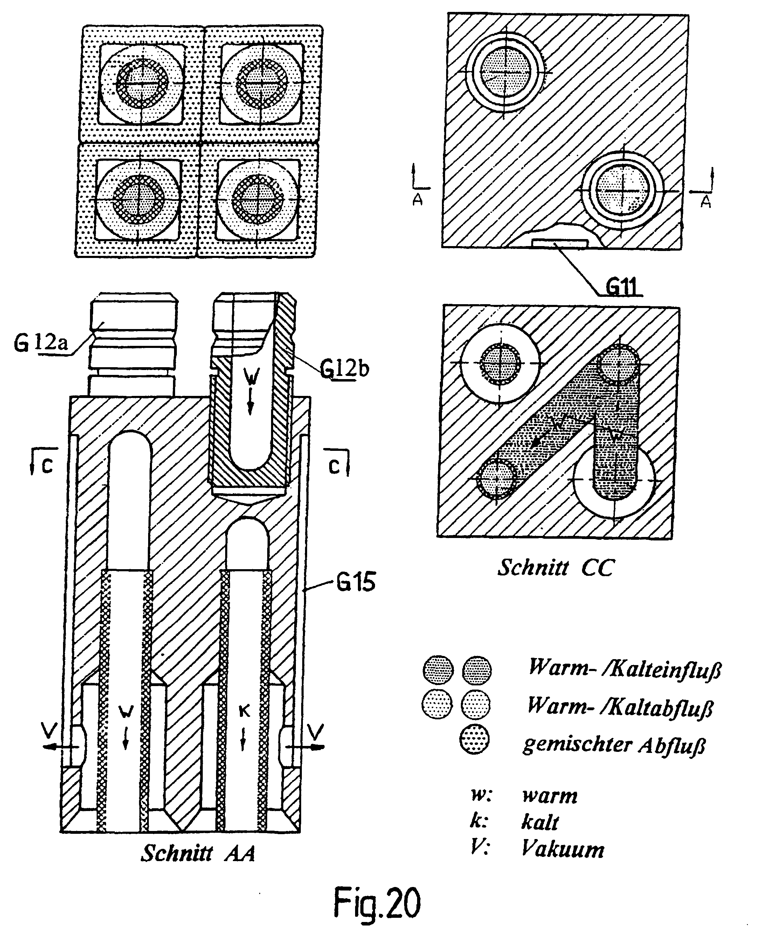

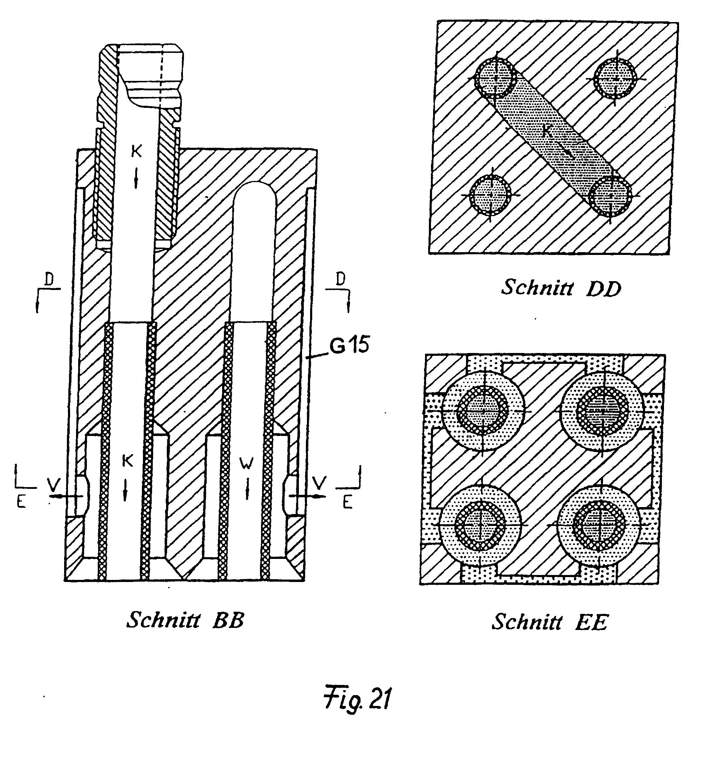

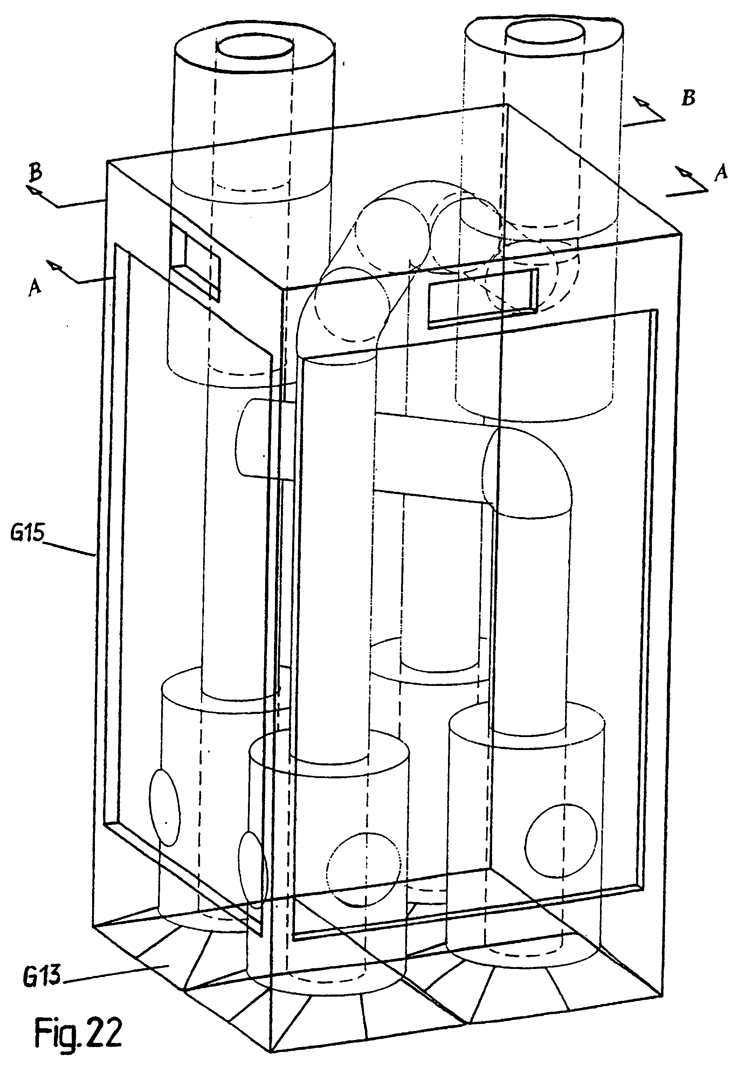

Die in den Figuren 18 bis 22 in ihren Einzelheiten stilisiert dargestellte Handbrause besteht aus einem Hohlkörper mit Zuführungskanälen G4 und Abzugskanälen G3. In dem Hohlkörper sind zwei übereinander in einem Abstand angeordnete Aufsätze G9 und G10 montiert, die miteinander mit Verbindungsschläuchen G8 gekoppelt sind.
Der Aufsatz G9 ist an den Innenwandungen des Hohlkörpers starr befestigt und weist in seinem Inneren zwei separate Zuführungskanalsysteme G4 zum Durchfluß von fluiden Stoffen auf. Jedes der Zuführungskanalsystem G4 ist mit gleicher Anzahl von senkrecht zur Stirnseite des Aufsatzes durchgehenden Bohrungen vom gleichen Querschnitt ausgestattet und derer Austrittsdüsen münden in einer Ebene der vorderen Stirnseite des Aufsatzes G9 ein. Die gegenüberliegenden Enden der Bohrungen der jeweiligen Kanalteilsysteme münden über Modulzuleitungen in einem der beiden zentralen Zuführungskanalsysteme ein, während die Einmündungsflächen in drei verschiedenen Ebenen innerhalb des Aufsatzes G10 liegen. Die hintere Stirnseite des Aufsatzes G9 ist mit drei Bohrungen größeren Querschnittes versehen, die über die zentralen Leitungsnetze mit den jeweiligen Kanalteilsystemen verbunden sind. Die Eingangsdüsen der Bohrungen sind über Flansche mit einen Zentralschlauch verbunden, über den eine entsprechende Versorgung mit fluiden Stoffen erfolgen kann.
Der Aufsatz G10 besteht aus zusammengefügten, gegeneinander beweglichen Modulen G15 aus einem wärmeisolierenden Material, die mit einer gleichen Anzahl von durchgehenden Bohrungen gleichen Querschnittes versehen sind. Die Bewegungsfreiheit der Module G15 gegeneinander ist durch die seitlich angeordneten Nuten G11, in denen sich je eine rollende Kugel befindet, eingeschränkt.
Jedes der Module G15 weist eine Anzahl der senkrecht zu seiner Vorderseite angeordneten und etwa in der Mitte abgesetzten Bohrungen auf, die je nach dem zuständigen Temperaturbereich Kanalsysteme, an eine der beiden Zentralanschlüsse G12a und G12b auf der Rückseite des Moduls G15 angeschlossen sind. Im unteren Bereich (Vorderseite) haben die abgesetzten Bohrungen einen größeren Durchmesser als hinter dem Absatz, der durch eine flache Abstufung ausgebildet ist. Jede dieser Bohrungen ist am Ausgang konisch unter einem Winkel von ca. 45 ° abgesetzt. In jede Bohrung wird ein Rohr bis zum Anschlag an der Abstufung eingesetzt und befestigt, wobei seine Abmaße so gewählt sind, daß der Durchfluß des fluiden Stoffes bis zur Haut hin und der Abfluß des Stoffes über die Abzugssammelräume G13 sich ungehindert vollziehen kann. Entlang der Seitenflächen jeden Moduls G15 sind Abzugsvertiefungen angeordnet, die unterhalb der Höhenabgrenzungen der Module liegen und über Querkanäle mit Bohrungen nach innen verbunden sind. Die Höhenlage der Abzugskanäle G4 ist so gewählt, daß sie ein gemeinsames Abzugsnetz mit der Einmündung in einen zentralen Abzugskanal bilden, wodurch die Absaugung des fluiden Stoffes nach außen erfolgen kann.

Am Umfang des Brausekopfes G2 kann eine Manschette in Form eines Dichtringes G6 aus einem flexiblen und/oder aus wärme- und/oder feuchteisolierendem Material befestigt werden, durch welche ein zumindest teilweise abgeschlossener Hohlraum zwischen Hautoberfläche und den Abzugskanälen G4 gebildet wird.

Die Abzugssammelräume G13 können untereinander verbunden sein und ein Minibad G14 bilden.

Die Reinigungsmittel haben im vorliegenden Ausführungsbeispiel eine Temperatur im Bereich von -30°C bis +60°C aufweisen, wobei warme Reinigungsmittel eine Temperatur zwischen +33 ± 0,1°C und +60°C und kalte Reinigungsmittel eine Temperatur zwischen -30°C ± 0,1°C und +33 ± 0,1°C besitzen.

Die hier beschriebene Erfindung ist nicht auf die dargestellten Ausführungsbeispiele beschränkt. Vielmehr ist es möglich, durch Variation der genannten Mittel und Merkmale weitere Ausführungsvarianten zu realisieren ohne den Rahmen der Erfindung zu verlassen.

### Bezugszeichenliste

- 1: Thermoblock
- 2: Elektroblock
- 3: Druckblock
- 3a: Verbindungsschlauch
- 4: Gehäuse
- 4a: Bohrung
- 4a': Bohrung
- 4a": Bohrung
- 5: Temperaturzone
- 5': n-Halbleiter
- 5a: Bohrung
- 5a': Bohrung
- 5a": Bohrung
- 6: Temperaturzone
- 6': p-Halbleiter
- 7: Bohrung
- 8: Bohrung
- 8a: Aufsatz
- 8a': Kanalleitung
- 9: Kanalsystem
- 9': Kanalsystem
- 9a: Modul
- 10: Sammelreservoir
- 10': Bohrung
- 10": Bohrung
- 11: Thermistor
- 11': Thermistor
- 11a: Kanalsystem
- 11b: Kanalsystem
- 12: Leitung
- 12': Leitung
- 12a: Zentrale Bohrung
- 12a': Verbindungskanal
- 12b: Zentrale Bohrung
- 12b': Verbindungskanal
- 13: Isolierstoff
- 13': Rohr
- 14: Überzug
- 14': Querkanal
- 14": Querkanal
- 15: Doppelfunktionale Pumpen
- 15': Abzugskanal
- 16: Meßverstärker
- 16a: Nuten
- 17: Reguliereinheit
- 19: Kühlsystem
- 19': Manschette
- 20: Pumpe
- 21: Poröses Kühlelement
- 21': Poröses Kühlelement
- 22: Tranpiratives Kühlsystem
- 22a: Polymerfilm
- 22b: Tränkfähige Schicht
- 22c: Hafthemmendes Pulver
- 23: Kühlkörper
- 24: Elektromechanisches Ventil
- 25: Elektromechanisches Ventil
- 26: Taktgeberblock
- 27: HF-Wechselstromgenerator
- 28: Versorgungseinheit
- 29: Digitales Out-Read
- G1: Handgriff
- G2: Brausekopf
- G2a: Zusatzteil
- G3: Abzugskanäle
- G4: Zuführungskanäle
- G5: Austrittsfläche
- G6: Dichtring
- G6a: Fasern
- G7: Ventil
- G7a: Feder
- G8: Verbindungsschlauch
- G9: erster Aufsatz
- G10: zweiter Aufsatz
- G11: Nut
- G12a: Zentralanschluß
- G12b: Zentralanschluß
- G13: Abzugssammelraum
- G14: Minibad
- G15: Modul
- G15: Modulunterseite
- G17: Begasungspumpe
- G18: Mischbatterie
- G19: Begasungseinrichtung
- G20: Befestigungs- und Höhenverstellungsring

## Patentansprüche

1. Verfahren zur nichtinvasiven Entnahme von Körperflüssigkeit über die Hautoberfläche, in dem die Hautschicht durchlässig gemacht wird, ohne sie irreversibel zu zerstören, wobei eng aneinander grenzende Bereiche der Hautoberfläche zeitgleich mit unterschiedlichen Temperaturen beaufschlagt werden und zusätzlich zeitlich definiert mit Unterdruck und gegebenenfalls zusätzlich mit wechselndem Überdruck druckbeaufschlagt werden.

2. Verfahren nach Anspruch 1, **dadurch gekennzeichnet, daß** die Bereiche der Hautoberfläche zeitlich definiert mit elektrischem Wechselstrom beaufschlagt werden.

3. Verfahren nach Anspruch 1 oder 2, **dadurch gekennzeichnet, daß** die Beaufschlagung der aneinander angrenzenden Bereiche der Hautoberfläche mit Temperatur und/oder Druck in einem vorgegebenen Zeittakt umgepolt wird.

4. Verfahren nach Anspruch 2, **dadurch gekennzeichnet, daß** die elektrische Wechselbeaufschlagung im Hochfrequenzbereich liegt.

5. Verfahren nach einem der Ansprüche 1 bis 4, **dadurch gekennzeichnet, daß** die aneinander angrenzenden Bereiche eine Flächenausdehnung von 1 mm² - 30 mm² aufweisen.

6. Verfahren nach Anspruch 5, **dadurch gekennzeichnet, daß** die aneinander angrenzenden Bereiche eine Flächenausdehnung von 4 mm² aufweisen.

7. Verfahren nach einem der Ansprüche 3 bis 6, **dadurch gekennzeichnet, daß** der Zeittakt der Umpolung im Bereich von 2 Sekunden bis 1800 Sekunden liegt.

8. Verfahren nach Anspruch 7, **dadurch gekennzeichnet, daß** der Zeittakt der Umpolung 10 Sekunden beträgt.

9. Verfahren nach Anspruch 4, **dadurch gekennzeichnet, daß** die Stromstärke des hochfrequenten Wechselstroms klein im Vergleich zu der kritischen Stromstärke ist, die die Schmerzgrenze des menschlichen Empfindens markiert.

10. Verfahren nach einem der Ansprüche 1 bis 9, **dadurch gekennzeichnet, daß** die Temperaturbeaufschlagung eine Wärme- und Kältebeaufschlagung umfaßt, wobei die Wärmebeaufschlagung im Temperaturbereich von +20°C bis +50°C und die Kältebeaufschlagung im Bereich von -15°C bis +20°C erfolgt.

11. Verfahren nach Anspruch 10, **dadurch gekennzeichnet, daß** die Wärmebeaufschlagung bei +44°C und die Kältebeaufschlagung bei -2°C erfolgt.

12. Verfahren nach einem der Ansprüche 1 bis 11, **dadurch gekennzeichnet, daß** die Druckbeaufschlagung ein Niederdruckvakuum im Bereich bis 1,0*10¹ Pa oder ein Überdruck im Bereich bis 1,0*10⁶ Pa ist.

13. Vorrichtung zur nichtinvasiven Entnahme von Körperflüssigkeit oder Injektion von Fluiden über die Hautoberfläche umfassend eine Thermoeinheit (1) zur Erzeugung von unterschiedlichen Temperaturen in aneinander angrenzenden Temperaturzonen (5, 6) einer Vorrichtungsoberfläche, welche mit der Hautoberfläche in Kontakt gebracht werden kann, und einer Druckeinheit (3) zur Erzeugung von gleichen oder unterschiedlichen Drücken in den Temperaturzonen, welche jeweils mit an der Vorrichtungsoberfläche mündenden Öffnungen (7, 8) über ein Kanalsystem (9, 9') mit der Druckeinheit (3) verbunden sind.

14. Vorrichtung nach Anspruch 13, **dadurch gekennzeichnet, daß** eine Elektroeinheit (2) zur Beaufschlagung der Hautoberfläche mit Wechselstrom vorgesehen ist, die eine Steuereinheit (26/16) aufweist.

15. Vorrichtung nach Anspruch 13 oder 14, **dadurch gekennzeichnet, daß** die Thermoeinheit (1), die Elektroeinheit (2) und die Druckeinheit (3) in einem Gehäuse (4) angeordnet sind, wobei die einzelnen Einheiten (1-3) mittels der Steuereinheit (26/16) nach einem vorgegebenen Programm funktionell gekoppelt sind.

16. Vorrichtung nach einem der Ansprüche 13 bis 15, **dadurch gekennzeichnet, daß** ein erstes Kanalsystem zur Beförderung warmer fluider Stoffe und ein zweites Kanalsystem zur Beförderung kalter fluider Stoffe vorgesehen ist und daß an den der Hautoberfläche zugewandten Öffnungen beider Kanalsysteme Abzugskanäle angeordnet sind, wobei das erste Kanalsystem und das zweite Kanalsystem mit einem Überdruck und die Abzugskanäle mit einem Unterdruck beaufschlagbar sind.

17. Vorrichtung nach einem der Ansprüche 14 oder 15, **dadurch gekennzeichnet, daß** die Thermoeinheit (1) mäanderförmige Peltier-Elemente mit dazwischen gefalteten n- und p-leitenden Halbleiterschenkeln (5',6') aufweist, wobei die Peltier-Elemente entweder seriell oder parallel durch die Steuereinheit mit Strom versorgbar sind.

18. Vorrichtung nach Anspruch 17, **dadurch gekennzeichnet, daß** die Peltier-Elemente mit ihren der Haut zugewandten Kontaktflächen in einer Kontaktebene liegen.

19. Vorrichtung nach Anspruch 18, **dadurch gekennzeichnet, daß** die wärmeleitenden Teile der Peltier-Elemente so gestaltet und/oder angesteuert sind, daß ihre Kontaktflächen gleich groß und in der Kontaktebene abwechselnd kalt/warm sind.

20. Vorrichtung nach einem der Ansprüche 17 bis 19, **dadurch gekennzeichnet, daß** die Peltier-Elemente mit Temperatursensoren zur Regulierung der eingestellten Temperaturgrenzwerte ausgestattet sind.

21. Vorrichtung nach Anspruch 20, **dadurch gekennzeichnet, daß** die Temperatursensoren Thermistoren sind.

22. Vorrichtung nach einem der Ansprüchen 13 bis 21, **dadurch gekennzeichnet, daß** die Druckeinheit (3) mit einem Sammelreservoir (10) zur Aufnahme bzw. Abgabe für fluide Stoffgemische verbunden ist, das Sammelreservoir (10) Verbindungen zu den Kanalöffnungen in den Kontaktflächen einerseits und zu einer Pumpe andererseits aufweist und daß für Kontrollzwecke und die Entsorgung ein zusätzlicher separater Zugang zum Sammelreservoir (10) vorhanden ist.

23. Vorrichtung nach einem der Ansprüche 17 bis 22, **dadurch gekennzeichnet, daß** die der Hautoberfläche zugewandten, von den Peltier-Elementen gebildeten Temperaturzonen (5,6) schachbrettartig angeordnet sind derart, daß warme und kalte Bereiche aneinandergrenzen.

24. Vorrichtung nach einem der Ansprüche 13 bis 23, **dadurch gekennzeichnet, daß** die Übergangsbereiche zwischen den warmen und kalten Temperaturzonen mit einem Isolierstoff (13) ausgefüllt sind.

25. Vorrichtung nach einem der Ansprüche 17 bis 23, **dadurch gekennzeichnet, daß** jedes Peltier-Element eine Bohrung (7, 8) aufweist, welche über ein Kanalsystem (9) und eine Pumpe (15) mit der Druckeinheit (3) verbunden ist.

26. Vorrichtung nach mindestens einem der Ansprüche 17 bis 25, **dadurch gekennzeichnet, daß** die Peltier-Elemente an der von der Hautoberfläche abgewandten Seite mit einem Kühlsystem (19) versehen sind, welches über die elektromechanischen Ventile (24, 25) an eine Pumpe (20) angeschlossen ist und eine zeitlich und räumlich separate Kühlung der momentan warm eingeschalteten Bereiche über die in ihm integrierten Kühlelemente (21, 21') gewährleistet.

27. Vorrichtung nach Anspruch 25, **dadurch gekennzeichnet, daß** die Arbeitsweise der als Saugund Druckpumpe betreibbaren Pumpe (15) über einen Taktgeber von der Steuereinheit (26) so steuerbar ist, daß je nach Bedarf dadurch ein Über- oder Unterdruck im Kanalsystem (9) erzeugbar ist.

28. Vorrichtung nach einem der Ansprüche 17 bis 27, **dadurch gekennzeichnet, daß** die Kühlung der Peltier-Elemente auf der der Hautoberfläche abgewandten Seite über ein geschlossenes transpiratives Kühlsystem (22) erfolgt.

29. Vorrichtung nach Anspruch 13, **dadurch gekennzeichnet, daß** eine Elektroeinheit (2) zur Beaufschlagung der Hautoberfläche mit Wechselstrom vorgesehen ist, die eine Steuereinheit (26/16) aufweist.

30. Vorrichtung nach Anspruch 29, **dadurch gekennzeichnet, daß** die Thermoeinheit (1), die Elektroeinheit (2) und die Druckeinheit (3) in einem Gehäuse (4) angeordnet sind, wobei die einzelnen Einheiten (1-3) mittels der Steuereinheit (26/16) nach einem vorgegebenen Programm funktionell gekoppelt sind.

31. Vorrichtung nach einem der Ansprüche 13 bis 30, **dadurch gekennzeichnet, daß** ein erstes Kanalsystem zur Beförderung warmer fluider Stoffe und ein zweites Kanalsystem zur Beförderung kalter fluider Stoffe vorgesehen ist und daß an den der Hautoberfläche zugewandten Öffnungen beider Kanalsysteme Abzugskanäle angeordnet sind, wobei das erste Kanalsystem und das zweite Kanalsystem mit einem Überdruck und die Abzugskanäle mit einem Unterdruck beaufschlagbar sind.

32. Vorrichtung nach einem der Ansprüche 29 oder 30, **dadurch gekennzeichnet, daß** die Thermoeinheit (1) mäanderförmige Peltier-Elemente mit dazwischen gefalteten n- und p-leitenden Halbleiterschenkeln (5',6') aufweist, wobei die Peltier-Elemente entweder seriell oder parallel durch die Steuereinheit mit Strom versorgbar sind.

33. Vorrichtung nach Anspruch 32, **dadurch gekennzeichnet, daß** die Peltier-Elemente mit ihren der Haut zugewandten Kontaktflächen in einer Kontaktebene liegen.

34. Vorrichtung nach Anspruch 33, **dadurch gekennzeichnet, daß** die wärmeleitenden Teile der Peltier-Elemente so gestaltet und/oder angesteuert sind, daß ihre Kontaktflächen gleich groß und in der Kontaktebene abwechselnd kalt/warm sind.

35. Vorrichtung nach einem der Ansprüche 32 bis 34, **dadurch gekennzeichnet, daß** die Peltier-Elemente mit Temperatursensoren zur Regulierung der eingestellten Temperaturgrenzwerte ausgestattet sind.

36. Vorrichtung nach Anspruch 35, **dadurch gekennzeichnet, daß** die Temperatursensoren Thermistoren sind.

37. Vorrichtung nach einem der Ansprüche 13 bis 36, **dadurch gekennzeichnet, daß** die Druckeinheit (3) mit einem Sammelreservoir (10) zur Aufnahme bzw. Abgabe für fluide Stoffgemische verbunden ist, das Sammelreservoir (10) Verbindungen zu den Kanalöffnungen in den Kontaktflächen einerseits und zu einer Pumpe andererseits aufweist und daß für Kontrollzwecke und die Entsorgung ein zusätzlicher separater Zugang zum Sammelreservoir (10) vorhanden ist.

38. Vorrichtung nach einem der Ansprüche 32 bis 37, **dadurch gekennzeichnet, daß** die der Hautoberfläche zugewandten, von den Peltier-Elementen gebildeten Temperaturzonen (5,6) schachbrettartig angeordnet sind derart, daß warme und kalte Bereiche aneinandergrenzen.

39. Vorrichtung nach einem der Ansprüche 13 bis 38, **dadurch gekennzeichnet, daß** die Übergangsbereiche zwischen den warmen und kalten Temperaturzonen mit einem Isolierstoff (13) ausgefüllt sind.

40. Vorrichtung nach einem der Ansprüche 32 bis 38, **dadurch gekennzeichnet, daß** jedes Peltier-Element eine Bohrung (7, 8) aufweist, welche über ein Kanalsystem (9) und eine Pumpe (15) mit der Druckeinheit (3) verbunden ist.

41. Vorrichtung nach mindestens einem der Ansprüche 32 bis 40, **dadurch gekennzeichnet, daß** die Peltier-Elemente an der von der Hautoberfläche abgewandten Seite mit einem Kühlsystem (19) versehen sind, welches über die elektromechanischen Ventile (24, 25) an eine Pumpe (20) angeschlossen ist und eine zeitlich und räumlich separate Kühlung der momentan warm eingeschalteten Bereiche über die in ihm integrierten Kühlelemente (21, 21') gewährleistet.

42. Vorrichtung nach Anspruch 40, **dadurch gekennzeichnet, daß** die Arbeitsweise der als Saugund Druckpumpe betreibbaren Pumpe (15) über einen Taktgeber von der Steuereinheit (26) so steuerbar ist, daß je nach Bedarf dadurch ein Über- oder Unterdruck im Kanalsystem (9) erzeugbar ist.

43. Vorrichtung nach einem der Ansprüche 32 bis 42, **dadurch gekennzeichnet, daß** die Kühlung der Peltier-Elemente auf der der Hautoberfläche abgewandten Seite über ein geschlossenes transpiratives Kühlsystem (22) erfolgt.

44. Vorrichtung nach Anspruch 13,
**dadurch gekennzeichnet, daß**
die nichtinvasive Entnahme von Körperflüssigkeit über die Hautoberfläche zur Reinigung des menschlichen Körpers dient, mit mindestens einem Brausekopf, wobei der Brausekopf Mittel zur Zuführung flüssiger Reinigungsmittel aufweist und zusätzlich zu den Mitteln zur Zuführung für die Reinigungsmittel Abzugskanäle angeordnet sind und die Mittel zur Zuführung mit einem Überdruck und die Abzugskanäle mit einem Unterdruck beaufschlagt sind und der Brausekopf zur Realisierung einer Thermokontrastdusche ein erstes Kanalsystem zur Beförderung warmer fluider Stoffe und ein zweites Kanalsystem zur Beförderung kalter fluider Stoffe direkt auf die Hautoberfläche aufweist und die Abzugskanäle an den Öffnungen beider Kanalsysteme angeordnet sind.

45. Vorrichtung nach Anspruch 44,
**dadurch gekennzeichnet, daß**
der Brausekopf aus zwei übereinander in einem Abstand angeordneten Aufsätzen (2a, 8a) besteht, die miteinander mit Verbindungsschläuchen (3a) gekoppelt sind.

46. Vorrichtung nach Anspruch 45,
**dadurch gekennzeichnet, daß**
der Aufsatz (8a) aus den zusammengefügten, gegeneinanderbeweglichen Modulen (9a) besteht, welche aus einem wärmeisolierenden Material gefertigt sind, und eine gleiche Anzahl von durchgehenden Bohrungen gleichen Querschnitts aufweisen, wobei die Bewegungsfreiheit der Module (9a) gegeneinander durch seitlich je zwei angeordnete Nuten (16a), in denen sich je eine rollende Kugel befindet, eingeschränkt ist.

47. Vorrichtung nach Anspruch 46,
**dadurch gekennzeichnet, daß**
jeder der Module (9a) eine Anzahl der senkrecht zu seiner Vorderseite angeordneten und in etwa in der Mitte abgesetzten Bohrungen (10' und 10") aufweist, die je nach dem zuständigen Temperaturbereich über eines der Kanalsysteme (11a und 11b) an eine der beiden Zentralbohrungen (12a und 12b) auf der Rückseite des Moduls angeschlossen sind.

48. Vorrichtung nach Anspruch 46 oder 47,
**dadurch gekennzeichnet, daß**
entlang der Seitenflächen jeden Moduls (9a) vierkantige Vertiefungen bzw. Abzugskanäle (15') angeordnet sind, die unterhalb der Höhenabgrenzungen der Module (9a) liegen und über die Querkanäle (14', 14") mit den Bohrungen (10', 10") nach innen verbunden sind, wobei die Höhenlage der Abzugskanäle so realisiert ist, daß sie ein gemeinsames Abzugsnetz mit der Einmündung in einen zentralen Abzugskanal (5a") bilden, wodurch die Absaugung des fluiden Stoffes nach außen erfolgen kann.

49. Vorrichtung nach Anspruch 44,
**dadurch gekennzeichnet, daß**
die Mittel zur Zuführung der flüssigen Reinigungsmittel Zuführungskanäle (G4) sind, welche ein Kanalsystem in einem Handgriff (G1) und/oder im Brausekopf (G2) bilden und die Abzugskanäle (G3) in unmittelbarer Nähe der Zuführungskanäle (G4) angeordnet sind.

50. Vorrichtung nach Anspruch 49,
**dadurch gekennzeichnet, daß**
an der Ein- und Austrittsfläche (G5) des Brausekopfes (G2) mindestens ein Abzugskanal (G3) taschenförmig oder ringförmig angeordnet ist und bei ringförmiger Anordnung mehrerer Abzugskanäle (G3) diese unterschiedliche Durchmesser aufweisen.

51. Vorrichtung nach Anspruch 49, **dadurch gekennzeichnet, daß**
an beziehungsweise in dem herkömmlichen Brausekopf ein separates Zusatzteil (G2a) befestigt ist, welches die Zuführungskanäle (G4) und/oder die Abzugskanäle (G3) aufweist.

52. Vorrichtung nach Anspruch 51,
**dadurch gekennzeichnet, daß**
an dem Brausekopf (G2) oder an dem Zusatzteil (G2a) ein als Manschette ausgebildeter Dichtring (G6) aus flexiblem Material, welcher zumindest teilweise einen als Minibad (G14) wirkenden abgeschlossenen Hohlraum zwischen Hautoberfläche und der Ein- und Austrittsfläche (G5) des Brausekopfes (G2) bildet, angeordnet ist.

53. Vorrichtung nach Anspruch 52,
**dadurch gekennzeichnet, daß**
der Dichtring (G6) aus einzelnen Fasern (G6a) oder Lamellen gebildet ist.

54. Vorrichtung nach Anspruch 53,
**dadurch gekennzeichnet, daß**
die Fasern (G6a) oder Lamellen unterschiedliche Gestalt mit rundem und/oder elyptischem und/oder rechteckigem Querschnitt aufweisen.

55. Vorrichtung nach Anspruch 53 oder 54,
**dadurch gekennzeichnet, daß**
die Fasern (G6a) oder Lamellen hohl ausgebildet sind und der Hohlraum durch einen Verbindungskanal (G3a) mit dem Abzugskanal (G3) zur Absaugung von Reinigungsmittel verbunden ist.

56. Vorrichtung nach mindestens einem der voranstehenden Ansprüche 49 bis 55,
**dadurch gekennzeichnet, daß**
die Zuführungskanäle (G4) Ventile aufweisen, welche bei Kontakt mit der Haut geöffnet werden und den Reinigungsmittelzufluß freigeben.

57. Vorrichtung nach mindestens einem der voranstehenden Ansprüche 49 bis 56,
**dadurch gekennzeichnet, daß**
der Zufluß des Reinigungsmittels über die Zuführungskanäle (G4) durch mindestens ein Ventil (G7) freigegeben oder gesperrt wird, wobei die Steuerung des Ventiles durch den Unterdruck in den Abzugskanälen (G3) erfolgt.

58. Vorrichtung nach mindestens einem der voranstehenden Ansprüche 49 bis 57,
**dadurch gekennzeichnet, daß**
die Zuführungskanäle (G4) und/oder die Abzugskanäle (G3) durch Bewegung der Module (G15) freigegeben beziehungsweise verschlossen werden.

59. Vorrichtung nach mindestens einem der voranstehenden Ansprüche 49 bis 58,
**dadurch gekennzeichnet, daß**
eine Steuervorrichtung die Unterdruckbeaufschlagung diskontinuierlich und/oder mit Unterbrechungen realisiert.

60. Vorrichtung nach mindestens einem der voranstehenden Ansprüche 49 bis 59,
**dadurch gekennzeichnet, daß**
die Unterseite (G15a) der Module (G15) konisch ausgebildet ist.

61. Vorrichtung nach Anspruch 16 oder 31,
**dadurch gekennzeichnet, daß**
die Mittel zur Zuführung der flüssigen Reinigungsmittel ein erstes Teilkanalsystem mit Zuführungskanälen (G4) zur Beförderung warmer fluider Stoffe und ein zweites Teilkanalsystem mit Zuführungskanälen (G4) zur Beförderung kalter fluider Stoffe aufweist und an den Öffnungen beider Teilkanalsysteme Abzugskanäle (G3) angeordnet sind, wobei das erste Teilkanalsystem und das zweite Teilkanalsystem mit einem Überdruck und die Abzugskanäle (G3) mit einem Unterdruck beaufschlagt sind.

62. Vorrichtung nach Anspruch 61,
**dadurch gekennzeichnet, daß**
entlang der Seitenflächen jeden Moduls (G15) Abzugsvertiefungen angeordnet sind, die unterhalb der Höhenabgrenzungen der Module (G15) liegen und über Querkanäle mit Bohrungen nach innen verbunden sind, wobei die Höhenlage der Abzugskanäle (G4) so realisiert ist, daß sie ein gemeinsames Abzugsnetz mit der Einmündung in einen zentralen Abzugskanal bilden, wodurch die Absaugung des fluiden Stoffes nach außen erfolgen kann.

63. Vorrichtung nach Anspruch 44,
**dadurch gekennzeichnet, daß**
die flüssigen Reinigungsmittel eine Temperatur im Bereich von -30°C bis +60°C aufweisen, wobei warme Stoffe bzw. Mittel eine Temperatur zwischen +33 ± 0,1°C und +60°C und kalte Stoffe bzw. Mittel eine Temperatur zwischen -30°C ± 0,1°C und +33 ± 0,1°C besitzen.

64. Vorrichtung nach Anspruch 44,
**dadurch gekennzeichnet, daß**
die Reinigung des menschlichen Körpers mit einer Handbrause, bestehend aus Handgriff und mindestens einem Brausekopf oder in einer Brausezelle mit mindestens einem automatisch geführten Brausekopf realisiert wird.

65. Vorrichtung nach Anspruch 44 oder 58,
**dadurch gekennzeichnet, daß**
die Module faltenbalgförmig ausgebildet sind derart, daß mehrere Faltenbalgkonstruktionen ineinandergeschachtelt angeordnet sind und hierdurch die Zuführungs- und Abzugskanäle gebildet werden.

66. Verfahren zur Reinigung des menschlichen Körpers mittels flüssiger Reinigungsmittel, wobei die Reinigungsmittel unter Erzeugung eines Unterdruckes auf der Hautoberfläche über Abzugskanäle abgesaugt werden,
**dadurch gekennzeichnet, daß**
beieinanderliegende Bereiche der Hautoberfläche über getrennte Kanäle zeitgleich mit warmen und kalten Reinigungsmitteln auf der Hautoberfläche beaufschlagt werden.

## Claims

1. Process for the non-intrusive take out of fluids through the skin surface by making the skin layer permeable without to destroy it, where contiguous areas of the skin surface are simultaneously subjected to different temperatures and, in addition, to timed periods to suction and possibly in addition to changing overpressure.

2. Process according to claim 1
**characterized** thereby that
the areas of the skin surface are subjected to timed periods of electrical alternating current.

3. Process according to claim 1 or 2
**characterized** thereby that
the application of the contiguous areas of the skin surface with temperature and/or pressure is switched over in a programmed time sequence.

4. Process according to claim 2
**characterized** thereby that
the electrical application lies in the high frequency range.

5. Process according to one of the claims 1 to 4
**characterized** thereby that
the contiguous areas amount to between 1 mm² and 30 mm².

6. Process according to claim 5
**characterized** thereby that
the continguous areas amount to 4 mm².

7. Process according to one of the claims 3 to 6
**characterized** thereby that
the duration of the phases lies between 2 and 1800 seconds.

8. Process according to claim 7
**characterized** thereby that
the duration of the phases is 10 seconds.

9. Process according to claim 4
**characterized** thereby that
the amperage of the high frequency alternating current is small in comparison to the critical power which defines the pain threshold of human sensation.

10. Process according to one of the claims 1 to 9
**characterized** thereby that
the temperature application includes an application of heat and cold whereby the heat application lies in the range from +20°C to +50°C and the cold application lies in the range of -15°C to +20°.

11. Process according to claim 10
**characterized** thereby that
heat application lies in the range of +44°C and the cold application lies in the range of -2°C.

12. Process according to one of the claims 1 to 11
**characterized** thereby that
the pressure application is a suction in the range up to 1.0* 10¹ Pa or an overpressure in the range up to 1.0* 10⁶.

13. Device for the non-intrusive take out of fluids through the skin surface which includes a heating unit (1) for simultaneous production of different temperatures in contiguous temperature zones (5, 6) of a device surface which can be brought into contact with the skin surface, and a pressure unit (3) for the production of equal or differing pressures in the temperature zones which are connected with openings (7, 8) at the surface of the device through a fluid transporting duct system (9, 9') with the pressure unit (3).

14. Device according to claim 13
**characterized** thereby that
an electrical unit (2) for applying alternating current to the skin surface which has a control unit (26/16).

15. Device according to claim 13 or 14
**characterized** thereby that
the heating unit (1), the electrical unit (2), and the pressure unit (3) are arranged in a housing (4), whereby the individual units (1 to 3) are functionally coupled by means of the control unit (26/16) according to a preset program.

16. Device according to one of the claims 13 to 15
**characterized** thereby that
a first duct system for transporting warm fluids and a second duct system for transporting cold fluids is provided, and that drainage ducts are placed at openings of both duct systems directed toward the skin surface whereby the first duct system and the second duct system are subjected to overpressure, and the drainage ducts are subjected to a suction.

17. Device according to one of the claims 14 or 15
**characterized** thereby that
the heating unit (1) has m-shaped Peltier elements with intermediately folded n- and p-conducting semiconductor legs (5', 6'), whereby the Peltier elements are either serially or parallel supplied with current by the control unit.

18. Device according to claim 17
**characterized** thereby that
the Peltier elements lie in a contact level with their contact surfaces facing toward the skin.

19. Device according to claim 18
**characterized** thereby that
the heat conducting parts of the Peltier elements are shaped and/or controlled so that their contact surfaces are equally large and in the contact level are alternately cold/warm.

20. Device according to one of the claims 17 to 19
**characterized** thereby that
the Peltier elements are equipped with temperature sensors for regulating the set temperature limit value levels.

21. Device according to claim 20
**characterized** thereby that
the temperature sensors are thermistors.

22. Device according to one of the claims 13 to 21
**characterized** thereby that
the pressure unit (3) is connected to a collection reservoir (10) for the supply of fluid mixtures, the collection reservoir (10) has connections to the duct openings in the contact surfaces on the one hand and to a pump on the other hand, and that an additional separate entrance to the collection reservoir (10) is present for control purposes.

23. Device according to one of the claims 17 to 22
**characterized** thereby that
the temperature zones (5, 6) formed by the Peltier elements and turned toward the skin surface are arranged like a chess board so that warm and cold areas are contiguous.

24. Device according to one of the claims 13 to 23
**characterized** thereby that
the transition areas between the warm and cold temperature zones are filled with an insulating material (13).

25. Device according to one of the claims 17 to 23
**characterized** thereby that
each Peltier element has a hole (7, 8) which is connected with the pressure unit (3) through a duct system (9) and a pump (15).

26. Device according to at least on of the claims 17 to 25
**characterized** thereby that
the Peltier elements are provided with a cooling system (19) on the side turned away from the skin surface which is connected to a pump (20) through solenoid valves (24, 25) and assures a chronologically and spatially separate cooling of the currently warmed areas by means of the cooling elements (21, 21') integrated into it.

27. Device according to claim 25
**characterized** thereby that
the working of the suction and positive pressure pump (15) can be controlled by the control unit (26) through a clock generator so that, according to need, an overpressure or a vacuum can be produced in the duct system (9).

28. Device according to one of the claims 17 to 27
**characterized** thereby that
the cooling of the Peltier elements on the side turned toward the skin surface takes place by means of a closed "sweating" cooling system (22) or heat exchanger.

29. Device according to claim 13
**characterized** thereby that
an electrical unit (2) for applying alternating current to the skin surface which has a control unit (26/16).

30. Device according to claim 29
**characterized** thereby that
the heating unit (1), the electrical unit (2), and the pressure unit (3) are arranged in a housing (4), whereby the individual units (1 to 3) are functionally coupled by means of the control unit (26/16) according to a preset program.

31. Device according to one of the claims 13 to 30
**characterized** thereby that
a first duct system for transporting warm fluids and a second duct system for transporting cold fluids is provided, and that drainage ducts are placed at openings of both duct systems directed toward the skin surface whereby the first duct system and the second duct system are subjected to overpressure, and the drainage ducts are subjected to a suction.

32. Device according to one of the claims 29 or 30
**characterized** thereby that
the heating unit (1) has m-shaped Peltier elements with intermediately folded n- and p-conducting semiconductor legs (5', 6'), whereby the Peltier elements are either serially or parallel supplied with current by the control unit.

33. Device according to claim 32
**characterized** thereby that
the Peltier elements lie in a contact level with their contact surfaces facing toward the skin.

34. Device according to claim 33
**characterized** thereby that
the heat conducting parts of the Peltier elements are shaped and/or controlled so that their contact surfaces are equally large and in the contact level are alternately cold/warm.

35. Device according to one of the claims 32 to 34
**characterized** thereby that
the Peltier elements are equipped with temperature sensors for regulating the set temperature limit value levels.

36. Device according to claim 35
**characterized** thereby that
the temperature sensors are thermistors.

37. Device according to one of the claims 13 to 36
**characterized** thereby that
the pressure unit (3) is connected to a collection reservoir (10) for the supply of fluid mixtures, the collection reservoir (10) has connections to the duct openings in the contact surfaces on the one hand and to a pump on the other hand, and that an additional separate entrance to the collection reservoir (10) is present for control purposes.

38. Device according to one of the claims 32 to 37
**characterized** thereby that
the temperature zones (5, 6) formed by the Peltier elements and turned toward the skin surface are arranged like a chess board so that warm and cold areas are contiguous.

39. Device according to one of the claims 13 to 38
**characterized** thereby that
the transition areas between the warm and cold temperature zones are filled with an insulating material (13).

40. Device according to one of the claims 32 to 38
**characterized** thereby that
each Peltier element has a hole (7, 8) which is connected with the pressure unit (3) through a duct system (9) and a pump (15).

41. Device according to at least on of the claims 32 to 40
**characterized** thereby that
the Peltier elements are provided with a cooling system (19) on the side turned away from the skin surface which is connected to a pump (20) through solenoid valves (24, 25) and assures a chronologically and spatially separate cooling of the currently warmed areas by means of the cooling elements (21, 21') integrated into it.

42. Device according to claim 40
**characterized** thereby that
the working of the suction and positive pressure pump (15) can be controlled by the control unit (26) through a clock generator so that, according to need, an overpressure or a vacuum can be produced in the duct system (9).

43. Device according to one of the claims 32 to 42
**characterized** thereby that
the cooling of the Peltier elements on the side turned toward the skin surface takes place by means of a closed "sweating" cooling system (22) or heat exchanger.

44. Device according to claim 13
**characterized** thereby that
the non-intrusive take out of fluids through the skin surface is made for cleaning the human body with at least one shower head, whereby the shower head has the means for the transport of wash water and, in addition to the means for applying wash water, drainage ducts are mounted, and the means for transport are subjected to overpressure and the drainage ducts are subject to a suction and the shower head for creating a thermal contrast shower has a first duct system for transporting warm fluids and a second duct system for transporting cold fluids directly to the skin surface, and the drainage ducts are mounted at the openings of both duct systems.

45. Device according to claim 44
**characterized** thereby that
the shower head consists of two attachments (2a, 8a) superimposed at some distance from each other and which are coupled to each other with connection hoses (3a).

46. Device according to claim 45
**characterized** thereby that
the attachment (8a) consists of fitted and moveable modules (9a) which are made of a heat insulating material and have an equal number of continuous holes of equal cross section, whereby the freedom of movement of the modules (9a) against each other is limited laterally by two mounted slots (16a) in each of which there is a rolling ball.

47. Device according to claim 46
**characterized** thereby that
each of the modules (9a) has a number of vertically (with respect to its front side) mounted and about in the middle offset holes (10' and 10") which, according to the responsible temperature area, is attached to one of the two central holes (12a and 12b) on the rear side of the module by means of one of the duct systems (11a and 11b).

48. Device according to claim 46 or 47
**characterized** thereby that
along the lateral sides of each module (9a) there are square holes or drainage ducts (15') which lie below the upper limit of the modules (9a) and are connected toward the inside with the holes (10' and 10") by means of the cross ducts (14' and 14"), whereby the height of the drainage ducts is arranged so that it forms a common drainage network with the infeed into a central drainage duct (5a"), through which the suction of the fluid toward the outside can take place.

49. Device according to claim 44
**characterized** thereby that
the means for transporting the wash water are supply ducts (G4) which form a duct system in a hand grip (G1) and/or in the shower head (G2) and the drainage ducts (G3) are located in the immediate vicinity of the supply ducts (G4).

50. Device according to claim 49
**characterized** thereby that
at least one pocket or ring shaped drainage duct (g3) is located at the inlet and outlet surfaces (G5) of the shower head (G2) and, in the case of a ring arrangement of several drainage ducts (G3), said ducts have different diameters.

51. Device according to claim 49
**characterized** thereby that
a separate attachment (G2a) is fastened in the traditional shower head which has the supply ducts (G4) and/or the drainage ducts (G3)

52. Device according to claim 51
**characterized** thereby that
at the shower head (G2) or at the separate attachment (G2a) a sleeve shaped sealing ring (G6) is fastened made of flexible material which forms a hollow space between the skin surface and the inlet and outlet surface (G5) of the shower head (G2) which at least partially functions as a mini-bath (G14).

53. Device according to claim 52
**characterized** thereby that
the sealing ring(G6) is made of individual fibers (G6a) or lamellas.

54. Device according to claim 53
**characterized** thereby that
the fibers (G6a) or lamellas are shaped differently with round and/or elliptical and/or square cross sections.

55. Device according to claim 53 or 54
**characterized** thereby that
the fibers (G6a) or lamellas form a hollow and the hollow space is connected through a connecting duct (G3a) with the drainage duct (G3) for the removal of wash water.

56. Device according to at least one of the claims 49 to 55 as above
**characterized** thereby that
the supply ducts (G4) have valves which open upon contact with the skin and release the wash water.

57. Device according to at least one of the claims 49 to 56 as above
**characterized** thereby that
the transport of the wash water through the supply ducts (G4) is released or blocked by at least one valve (G7), whereby the control of the valves takes place by means of the suction in the drainage ducts (G4).

58. Device according to at least one of the claims 49 to 57 as above
**characterized** thereby that
the supply ducts (G4) and/or the drainage ducts (G3) are released or closed by means of the movement of the modules (G15).

59. Device according to at least one of the claims 49 to 58 as above
**characterized** thereby that
a control device which produces the suction discontinuously and/or with interruptions.

60. Device according to at least one of the claims 49 to 59 as above
**characterized** thereby that
the lower side (G15a) of the module (G15) is conically shaped.

61. Device according to claim 16 or 31
**characterized** thereby that
the means for transport of the wash water has a first partial duct system with supply ducts (G4) for supplying warm fluids and a second partial duct system with supply ducts (G4) for supply cold fluids and drainage ducts (G3) are placed at the openings of both partial ducts systems whereby the first partial duct system and the second partial duct system are subjected to overpressure and the drainage ducts (G3) are subjected to suction.

62. Device according to claim 61
**characterized** thereby that
along the lateral surfaces of each module (G15) there are drainage holes which lie below the upper limits of the modules (G15) and are connected by means of cross ducts with holes toward the inside, whereby the height of the drainage ducts (G4) is arranged so that they form a common drainage network through the removal of the fluid toward the outside can take place.

63. Device according to claim 44
**characterized** thereby that
the wash water has a temperature in the range from -30°C to +60°C, whereby warm materials or means have a temperature between +33 ± 0.1°C and +60°C and cold materials or means have a temperature between -30°C and +33 ± 0.1 °C.

64. Device according to claim 44
**characterized** thereby that
the cleaning of the human body is carried out with a hand shower which consists of a hand grip and at least one shower head or in a shower stall with at least one automatically controlled shower head.

65. Device according to claim 44 or 58
**characterized** thereby that
the modules are formed as folding bellows so that several folding bellow constructions are fitted into each other and thus form supply and drainage ducts.

66. Process for cleaning the human body by means of wash water whereby the wash water is removed from the skin surface through drainage ducts under negative pressure
**characterized** thereby that
contiguous areas of the skin surface are simultaneously subjected to hot and cold wash water through separate ducts on the skin surface.

## Revendications

1. Procédé d'enlèvement non traumatique de liquide biologique sur la surface de la peau, dans lequel la couche dermique est rendue perméable, sans la détruire de manière irréversible, des zones étroitement adjacentes de la surface de la peau étant en même temps sollicitées à des températures différentes et de plus sollicitées par une dépression pendant un temps défini et éventuellement de plus sollicitées par une surpression variable.

2. Procédé selon la revendication 1, **caractérisé en ce que** les zones de la surface de la peau sont sollicitées pendant un temps défini par un courant électrique alternatif.

3. Procédé selon la revendication 1 ou 2, **caractérisé en ce que** la sollicitation des zones adjacentes de la surface de la peau par température et/ou pression fait l'objet d'un changement de polarisation dans un signal de rythme prédéfini.

4. Procédé selon la revendication 2, **caractérisé en ce que** la sollicitation électrique au courant alternatif se situe dans la zone de haute fréquence.

5. Procédé selon une des revendications 1 à 4, **caractérisé en ce que** les zones adjacentes présentent une extension de surface de 1 mm² à 30 mm².

6. Procédé selon la revendication 5, **caractérisé en ce que** les zones adjacentes présentent une extension de surface de 4 mm².

7. Procédé selon une des revendications 3 à 6, **caractérisé en ce que** le signal de rythme du changement de polarisation se situe dans une plage de 2 secondes à 1800 secondes.

8. Procédé selon la revendication 7, **caractérisé en ce que** le signal de rythme du changement de polarisation est d'une durée de 10 secondes.

9. Procédé selon la revendication 4, **caractérisé en ce que** la puissance du courant alternatif à haute fréquence est faible comparativement à la puissance critique du courant qui marque la limite de douleur de la sensibilité humaine.

10. Procédé selon une des revendications 1 à 9, **caractérisé en ce que** la sollicitation par température englobe une sollicitation par la chaleur et le froid, la sollicitation par la chaleur ayant lieu dans une plage de température de +20°C à +50°C et la sollicitation par le froid dans une plage de -15°C à + 20°C.

11. Procédé selon la revendication 10, **caractérisé en ce que** la sollicitation par la chaleur a lieu à +44°C et que la sollicitation par le froid a lieu à -2°C.

12. Procédé selon une des revendications 1 à 11, **caractérisé en ce que** la sollicitation par pression est un vide par basse pression dans une plage allant jusqu'à 1,0*10¹ Pa ou une surpression dans une plage allant jusqu'à 1,0*10⁶ Pa.

13. Dispositif d'enlèvement non traumatique de liquide biologique ou d'injection de fluides sur la surface de la peau comportant une unité thermique (1) pour la création de températures différentes dans des zones de température adjacentes (5, 6) d'une surface du dispositif qui peut être mise en contact avec la surface de la peau, et une unité de pression (3) pour la création de pressions égales ou différentes dans les zones de température, qui sont respectivement reliées avec l'unité de pression (3) par des orifices (7, 8) débouchant sur la surface du dispositif par l'intermédiaire d'un système de canaux (9, 9').

14. Dispositif selon la revendication 13, **caractérisé en ce qu'**il est prévu une unité électrique (2) pour la sollicitation de la surface de la peau au courant alternatif qui présente une unité de commande (26/16).

15. Dispositif selon la revendication 13 ou 14, **caractérisé en ce que** l'unité thermique (1), l'unité électrique (2) et l'unité de pression (3) sont disposées dans un boîtier (4), les différentes unités (1 - 3) étant couplées au niveau fonctionnel au moyen de l'unité de commande (26/16) suivant un programme prédéfini.

16. Dispositif selon une des revendications 13 à 15, **caractérisé en ce qu'**il est prévu un premier système de canaux pour le transport de substances fluides chaudes et un deuxième système de canaux pour le transport de substances fluides froides et qu'aux orifices tournés vers la surface de la peau des deux systèmes de canaux, des canaux d'évacuation sont disposés, le premier système de canaux et le deuxième système de canaux pouvant être sollicités par une surpression et les canaux d'évacuation par une dépression.

17. Dispositif selon une des revendications 14 ou 15, **caractérisé en ce que** l'unité thermique (1) présente des éléments Peltier en forme de méandres avec des branches à semi-conducteurs conductrices n et p (5', 6') pliées dans l'intervalle, les éléments Peltier pouvant être alimentés en courant soit en série, soit en parallèle, par l'unité de commande.

18. Dispositif selon la revendication 17, **caractérisé en ce que** les éléments Peltier avec leurs surfaces de contact tournées vers la peau se situent dans un plan de contact.

19. Dispositif selon la revendication 18, **caractérisé en ce que** les pièces conductrices de chaleur des éléments Peltier sont configurées et/ou commandées de manière' à ce que leurs surfaces de contact soient de dimensions identiques et alternativement froides/chaudes dans le plan de contact.

20. Dispositif selon une des revendications 17 à 19, **caractérisé en ce que** les éléments Peltier sont équipés de capteurs de température pour la régulation des valeurs limites de température fixées.

21. Dispositif selon la revendication 20, **caractérisé en ce que** les capteurs de température sont des thermistors.

22. Dispositif selon une des revendications 13 à 21, **caractérisé en ce que** l'unité de pression (3) est reliée avec un réservoir collecteur (10) servant à recevoir ou à distribuer des mélanges fluides de substances, que le réservoir collecteur (10) présente des liaisons avec les ouvertures des canaux dans les surfaces de contact d'une part et avec une pompe d'autre part et qu'un accès séparé supplémentaire au réservoir collecteur (10) est prévu à des fins de contrôle et d'élimination.

23. Dispositif selon une des revendications 17 à 22, **caractérisé en ce que** les zones de température (5, 6) formées par les éléments Peltier tournées vers la surface de la peau sont disposées à la manière d'un échiquier de manière à ce que les zones chaudes et froides soient adjacentes.

24. Dispositif selon une des revendications 13 à 23, **caractérisé en ce que** les zones de transition entre les zones de température chaudes et froides sont remplies par une matière isolante (13).

25. Dispositif selon une des revendications 17 à 23, **caractérisé en ce que** chaque élément Peltier présente un alésage (7, 8) qui est relié par l'intermédiaire d'un système de canaux (9) et d'une pompe (15) avec l'unité de pression (3).

26. Dispositif selon au moins une des revendications 17 à 25, **caractérisé en ce que** les éléments Peltier sont pourvus, sur leur face détournée de la surface de la peau, d'un système de refroidissement (19) qui est raccordé par l'intermédiaire des soupapes électromécaniques (24, 25) à une pompe (20) et garantit un refroidissement séparé dans le temps et dans l'espace des zones momentanément sollicitées par chaleur par l'intermédiaire des éléments de refroidissement (21, 21') qui y sont intégrés.

27. Dispositif selon la revendication 25, **caractérisé en ce que** le mode de fonctionnement de la pompe (15) utilisable comme pompe d'aspiration et de refoulement peut être commandé par l'intermédiaire d'un générateur d'impulsions par l'unité de commande (26) de manière à ce qu'une surpression ou une dépression puisse être créée dans le système de canaux (9) en fonction des besoins.

28. Dispositif selon une des revendications 17 à 27, **caractérisé en ce que** le refroidissement des éléments Peltier sur la face détournée de la surface de la peau est réalisé grâce à un système transpiratif fermé de refroidissement (22).

29. Dispositif selon la revendication 13, **caractérisé en ce qu'**il est prévu une unité électrique (2) pour la sollicitation de la surface de la peau au courant alternatif qui présente une unité de commande (26/16).

30. Dispositif selon la revendication 29, **caractérisé en ce que** l'unité thermique (1), l'unité électrique (2) et l'unité de pression (3) sont disposées dans un boîtier (4), les différentes unités (1 - 3) étant couplées au niveau fonctionnel au moyen de l'unité de commande (26/16) suivant un programme prédéfini.

31. Dispositif selon une des revendications 13 à 30, **caractérisé en ce qu'**il est prévu un premier système de canaux pour le transport de substances fluides chaudes et un deuxième système de canaux pour le transport de substances fluides froides et qu'aux orifices tournés vers la surface de la peau des deux systèmes de canaux, des canaux d'évacuation sont disposés, le premier système de canaux et le deuxième système de canaux pouvant être sollicités par une surpression et les canaux d'évacuation par une dépression.

32. Dispositif selon une des revendications 29 ou 30, **caractérisé en ce que** l'unité thermique (1) présente des éléments Peltier en forme de méandres avec des branches à semi-conducteurs conductrices n et p (5', 6') pliées dans l'intervalle, les éléments Peltier pouvant être alimentés en courant soit en série, soit en parallèle, par l'unité de commande.

33. Dispositif selon la revendication 32, **caractérisé en ce que** les éléments Peltier avec leurs surfaces de contact tournées vers la peau se situent dans un plan de contact.

34. Dispositif selon la revendication 33, **caractérisé en ce que** les pièces conductrices de chaleur des éléments Peltier sont configurées et/ou commandées de manière à ce que leurs surfaces de contact soient de dimensions identiques et alternativement froides/chaudes dans le plan de contact.

35. Dispositif selon une des revendications 32 à 34, **caractérisé en ce que** les éléments Peltier sont équipés de capteurs de température pour la régulation des valeurs limites de températures fixées.

36. Dispositif selon la revendication 35, **caractérisé en ce que** les capteurs de température sont des thermistors.

37. Dispositif selon une des revendications 13 à 36, **caractérisé en ce que** l'unité de pression (3) est reliée avec un réservoir collecteur (10) servant à recevoir ou à distribuer des mélanges fluides de substances, que le réservoir collecteur (10) présente des liaisons avec les ouvertures des canaux dans les surfaces de contact d'une part et avec une pompe d'autre part et qu'un accès séparé supplémentaire au réservoir collecteur (10) est prévu à des fins de contrôle et d'élimination.

38. Dispositif selon une des revendications 32 à 37, **caractérisé en ce que** les zones de température (5, 6) formées par les éléments Peltier tournées vers la surface de la peau sont disposées à la manière d'un échiquier de manière à ce que des zones chaudes et froides soient adjacentes.

39. Dispositif selon une des revendications 13 à 38, **caractérisé en ce que** les zones de transition entre les zones de température chaudes et froides sont remplies par une matière isolante (13).

40. Dispositif selon une des revendications 32 à 38, **caractérisé en ce que** chaque élément Peltier présente un alésage (7, 8) qui est relié par l'intermédiaire d'un système de canaux (9) et d'une pompe (15) avec l'unité de pression (3).

41. Dispositif selon au moins une des revendications 32 à 40, **caractérisé en ce que** les éléments Peltier sont pourvus, sur leur face détournée de la surface de la peau, d'un système de refroidissement (19) qui est raccordé par l'intermédiaire des soupapes électromécaniques (24, 25) à une pompe (20) et garantit un refroidissement séparé dans le temps et dans l'espace des zones momentanément sollicitées par chaleur par l'intermédiaire des éléments de refroidissement (21, 21') qui y sont intégrés.

42. Dispositif selon la revendication 40, **caractérisé en ce que** le mode de fonctionnement de la pompe (15) utilisable comme pompe d'aspiration et de refoulement peut être commandé par l'intermédiaire d'un générateur d'impulsions par l'unité de commande (26) de manière à ce qu'une surpression ou une dépression puisse être créée dans le système de canaux (9) en fonction des besoins.

43. Dispositif selon une des revendications 32 à 42, **caractérisé en ce que** le refroidissement des éléments Peltier a lieu sur la face détournée de la surface de la peau par l'intermédiaire d'un système transpiratif fermé de refroidissement (22).

44. Dispositif selon la revendication 13, **caractérisé en ce que** l'enlèvement non traumatique de liquide biologique sur la surface de la peau sert au nettoyage du corps humain avec au moins une tête d'aspersion, la tête d'aspersion présentant des moyens pour l'acheminement de produit nettoyant liquide et que des canaux d'évacuation sont disposés en plus des moyens d'acheminement pour le produit nettoyant et que les moyens d'acheminement sont sollicités par une surpression et les canaux d'évacuation par une dépression et que la tête d'aspersion présente, afin de procéder à une douche à contraste thermique, un premier système de canaux pour transporter des substances fluides chaudes et un deuxième système de canaux pour transporter des substances fluides froides directement sur la surface de la peau et que les canaux d'évacuation sont disposés aux ouvertures des deux systèmes de canaux.

45. Dispositif selon la revendication 44, **caractérisé en ce que** la tête d'aspersion consiste en deux garnitures (2a, 8a) disposées à distance l'une au dessus de l'autre, qui sont couplées l'une avec l'autre par des tuyaux de liaison (3a).

46. Dispositif selon la revendication 45, **caractérisé en ce que** la garniture (8a) est composée des modules assemblés réciproquement mobiles en sens inverse (9a) qui sont fabriqués dans une matière isolatrice thermique et présentent un nombre égal d'alésages de même section transversale les traversant, la liberté de mouvement des modules (9a) l'un vis-à-vis de l'autre étant restreinte par à chaque fois deux rainures (16a) disposées latéralement, dans lesquelles se trouve à chaque fois une bille roulante.

47. Dispositif selon la revendication 46, **caractérisé en ce que** chacun des modules (9a) présente un certain nombre d'alésages (10' et 10' ') disposés à la verticale de sa face frontale et un peu décalés au centre qui sont raccordés, en fonction de la zone de température active, par l'intermédiaire d'un des systèmes de canaux (11a et 11b) à un des deux alésages centraux (12a et 12b) sur la face arrière du module.

48. Dispositif selon la revendication 46 ou 47, **caractérisé en ce que** sont disposés, le long des surfaces latérales de chaque module (9a), des renfoncements carrés ou des canaux d'évacuation (15') qui se trouvent en dessous des limitations en hauteur des modules (9a) et sont reliés vers l'intérieur par l'intermédiaire des canaux transversaux (14', 14'') avec les alésages (10', 10"), le positionnement en hauteur des canaux d'évacuation étant réalisé de manière à ce qu'ils forment avec l'embouchure vers un canal central d'évacuation (5a'') un réseau commun d'évacuation, ce qui permet d'assurer l'aspiration vers l'extérieur de la substance fluide.

49. Dispositif selon la revendication 44, **caractérisé en ce que** les moyens d'acheminement du produit nettoyant liquide sont des canaux d'acheminement (G4) qui forment un système de canaux dans une poignée (G1) et/ou une tête d'aspersion (G2) et que les canaux d'évacuation (G3) sont disposés à proximité immédiate des canaux d'acheminement (G4).

50. Dispositif selon la revendication 49, **caractérisé en ce que**, sur la surface d'entrée et de sortie (G5) de la tête d'aspersion (G2), au moins un canal d'évacuation (G3) est disposé en forme de poche ou d'anneau et qu'en cas de disposition en anneau de plusieurs canaux d'évacuation (G3), ceux-ci présentent des diamètres différents.

51. Dispositif selon la revendication 49, **caractérisé en ce que**, sur ou dans la tête d'aspersion traditionnelle, est fixée une pièce ajoutée séparée (G2a) qui présente les canaux d'acheminement (G4) et/ou les canaux d'évacuation (G3).

52. Dispositif selon la revendication 51, **caractérisé en ce que**, sur la tête d'aspersion (G2) ou sur la pièce ajoutée (G2a), est disposée une bague d'étanchéité (G6) configurée comme un manchon en matériau souple, qui forme du moins partiellement une cavité fermée agissant comme un mini-bain (G14) entre la surface de la peau et la surface d'entrée et de sortie (G5) de la tête d'aspersion (G2).

53. Dispositif selon la revendication 52, **caractérisé en ce que** la bague d'étanchéité (G6) est constituée de fibres (G6a) individuelles ou de lamelles.

54. Dispositif selon la revendication 53, **caractérisé en ce que** les fibres (G6a) ou les lamelles présentent des formes différentes avec une section transversale ronde et/ou elliptique et/ou rectangulaire.

55. Dispositif selon la revendication 53 ou 54, **caractérisé en ce que** les fibres (G6a) ou les lamelles sont d'une configuration creuse et que la cavité est reliée par l'intermédiaire d'un canal de liaison (G3a) avec le canal d'évacuation (G3) pour l'aspiration de produit nettoyant.

56. Dispositif selon au moins une des revendications précédentes 49 à 55, **caractérisé en ce que** les canaux d'acheminement (G4) présentent des soupapes qui sont ouvertes en cas de contact avec la peau et libèrent l'afflux de produit nettoyant.

57. Dispositif selon au moins une des revendications précédentes 49 à 56, **caractérisé en ce que** l'afflux de produit nettoyant par les canaux d'acheminement (G4) est libéré ou bloqué par au moins une soupape (G7), la commande de la soupape étant assurée grâce à la dépression dans les canaux d'évacuation (G3).

58. Dispositif selon au moins une des revendications précédentes 49 à 57, **caractérisé en ce que** les canaux d'acheminement (G4) et/ou les canaux d'évacuation (G3) sont libérés ou fermés par un mouvement des modules (G15).

59. Dispositif selon au moins une des revendications précédentes 49 à 58, **caractérisé en ce qu'**un dispositif de commande assure de manière discontinue et/ou avec des interruptions la sollicitation par dépression.

60. Dispositif selon au moins une des revendications précédentes 49 à 59, **caractérisé en ce que** la face inférieure (G15a) des modules (G15) est de forme conique.

61. Dispositif selon la revendication 16 ou 31, **caractérisé en ce que** les moyens d'acheminement de produit nettoyant liquide présentent un premier système de canaux partiel avec des canaux d'acheminement (G4) pour le transport de substances fluides chaudes et un deuxième système de canaux partiel avec des canaux d'acheminement (G4) pour le transport de substances fluides froides et que des canaux d'évacuation (G3) sont disposés aux ouvertures des deux systèmes de canaux partiels, le premier système de canaux partiel et le deuxième système de canaux partiel étant sollicités par une surpression et les canaux d'évacuation (G3) par une dépression.

62. Dispositif selon la revendication 61, **caractérisé en ce que** sont disposés, le long des surfaces latérales de chaque module (G15), des renfoncements d'évacuation qui sont situés en dessous des limitations en hauteur des modules (G15) et sont reliés vers l'intérieur avec des alésages par l'intermédiaire de canaux transversaux, le positionnement en hauteur des canaux d'évacuation (G4) étant réalisé de manière à ce qu'ils forment avec l'embouchure vers un canal central d'évacuation un réseau commun d'évacuation, ce qui permet d'assurer l'aspiration vers l'extérieur de la substance fluide.

63. Dispositif selon la revendication 44, **caractérisé en ce que** les produits nettoyants liquides présentent une température dans la plage de -30°C à +60°C, les substances ou produits chauds ayant une température située entre +33 ± 0,1°C et +60°C et les substances ou produits froids ayant une température située entre -30°C ± 0,1°C et +33 ± 0,1°C.

64. Dispositif selon la revendication 44, **caractérisé en ce que** le nettoyage du corps humain est réalisé à l'aide d'un dispositif manuel d'aspersion, consistant en une poignée et au moins une tête d'aspersion ou en une cellule d'aspersion comportant au moins une tête d'aspersion guidée automatiquement.

65. Dispositif selon la revendication 44 ou 58, **caractérisé en ce que** les modules sont configurés comme des soufflets de manière à ce que plusieurs ensembles à soufflets soient disposés emboîtés les uns dans les autres et qu'ainsi des canaux d'acheminement et d'évacuation soient constitués.

66. Procédé pour le nettoyage du corps humain au moyen de produits nettoyants liquides, les produits nettoyants étant aspirés par des canaux d'évacuation en créant une dépression sur la surface de la peau, **caractérisé en ce que** des zones adjacentes de la surface de la peau sont sollicitées par l'intermédiaire de canaux séparés en même temps par des produits nettoyants chauds et froids appliqués sur la surface de la peau.
